# EUROPÄISCHE PATENTSCHRIFT

(11) **EP 2 744 468 B1**
(45) Veröffentlichungstag und Bekanntmachung des Hinweises auf die Patenterteilung: **03.06.2015**
(21) Anmeldenummer: 12748210.7
(22) Anmeldetag: 15.08.2012
(51) Int. Cl.: A61M 39/10, A61J 1/20, A61M 5/162

(54) **ADAPTER FÜR EINE TRANSFERVORRICHTUNG FÜR EIN FLUID SOWIE TRANSFERVORRICHTUNG**
ADAPTER FOR A TRANSFER DEVICE FOR A FLUID, AND TRANSFER DEVICE
ADAPTATEUR POUR UN DISPOSITIF DE TRANSFERT DE FLUIDE ET DISPOSITIF DE TRANSFERT

(30) Priorität: 18.08.2011 EP 11177918
(43) Veröffentlichungstag der Anmeldung: 25.06.2014
(73) Patentinhaber: Weibel CDS AG, 9104 Waldstatt (CH)
(72) Erfinder: WEIBEL, Ludwig, Daniel, CH-9104 Waldstatt (CH)
(74) Vertreter: Hepp Wenger Ryffel AG
(86) Internationale Anmeldenummer: PCT/EP2012/065955
(87) Internationale Veröffentlichungsnummer: WO 2013/024120

(56) Entgegenhaltungen:
- WO-A1-01/00261
- FR-A1- 2 509 689
- FR-A1- 2 753 624
- US-A- 4 253 501

## Beschreibung

Die Erfindung betrifft einen Adapter für eine Transfervorrichtung für ein Fluid, zum Anschluss eines Behälters enthaltend das Fluid, gemäss dem Oberbegriff des Anspruchs 1. Die Erfindung betrifft weiter eine aus derartigen Adaptern gebildete Transfervorrichtung für ein Fluid aus einem Behälter zu einer Abgabevorrichtung.

Bei derartigen Transfervorrichtungen handelt es sich in der Regel um Vorrichtungen für die nadellose Entnahme von pharmazeutischen Substanzen aus einem oder mehreren hermetisch verschlossenen Glasgefäss(en). Insbesondere ist es häufig erforderlich, verschiedene medizinische Substanzen (z.B. flüssig/flüssig oder flüssig/fest) erst kurz vor der Verabreichung miteinander zu vermischen bzw. ineinander aufzulösen, da diese in Mischung bzw. in Lösung nicht lagerfähig sind. Hierzu müssen die verschiedenen medizinischen Substanzen in getrennten Behältern aufbewahrt und unter sterilen Bedingungen gemischt werden, bevor sie in eine Abgabevorrichtung z.B. eine Injektionsspritze transferiert werden. Derartige Vorrichtungen können selbstverständlich auch im nichtmedizinischen Bereich Anwendung finden, beispielsweise für chemische Analysezwecke im Lebensmittelbereich oder im Kosmetikbereich.

Bekannte Vorrichtungen zum Mischen und Verabreichen von zwei medizinischen Substanzen umfassen Doppelkammerspritzen wie beispielsweise in der US 4,424,057 beschrieben. Diese können in gebrauchsfertigem Zustand zur einmaligen Benutzung abgepackt werden, haben aber den Nachteil, dass sie eine vergleichsweise grosse Baulänge haben und daher schwierig zu lagern sind. Ausserdem kann nicht auf Medikamentenbehälter mit genormten Anschlusskupplungen zurückgegriffen werden, sondern die Spritze muss individuell befüllt werden.

WO 2009/146088 A1 beschreibt einen Adapter einer Transfervorrichtung für ein Fluid, welcher zum Mischen zweier medizinischer Substanzen den Anschluss von zwei genormten Medikamentenbehältern ermöglicht, welche mit einem penetrierbaren Verschlusselement z.B. aus Gummi verschlossen sind. Der Adapter weist hierzu zwei entgegengesetzt angeordnete Kopplungsstellen für die Behälter mit jeweils wenigstens einer Hohlnadel zum Durchstechen des Verschlusselements auf. Die Behälter werden dabei in einen Rastkragen der Kopplungsstellen eingeschnappt, wobei die Hohlnadel das Verschlusselement durchdringt. Die Hohlnadeln können über Fluidkanäle des Adapters miteinander verbunden werden, um die Medikamente zu mischen. Über einen Schieber kann die Verbindung zu einem Anschlussport hergestellt werden, an welchem eine Spritze zum Aufziehen des Gemischs angeschlossen werden kann.

WO 2008 /126090 A1 beschreibt eine Transfervorrichtung für ein flüssiges Medikament, welches vor der Verabreichung mit einem festen oder flüssigen Medikament vermischt werden muss. Hierzu ist eine Kopplungsstelle mit einer Hohlnadel für einen mit einem penetrierbaren Verschlusselement verschlossenen Behälter mit einem flüssigen oder festen Medikament vorgesehen. Ein weiteres flüssiges Medikament wird von einer Spritze an einem Anschlussport eingebracht und gelangt über Fluidkanäle des Adapters in den Behälter. Ein weiterer Port dient der Abgabe des gemischten Medikaments. Ein Sperrhahn ermöglicht den fluiddichten Abschluss der Hohlnadel, um ein Abtropfen des Medikamentengemischs zu verhindern, wenn der Behälter abgenommen bzw. gewechselt wird. FR 2 753 624 A1 beschreibt die Verbindung zwischen einem ersten Gefäß mit Hals, das mit einem viskoelastischen, durchstechbaren Stopfen verschlossen ist, einerseits und einem zweiten, mit einem aktiven Anschluss versehenen Gefäß andererseits.

Ein erheblicher Nachteil bekannter Transfervorrichtungen mit einem Adapter besteht darin, dass die Behälter erst kurz vor dem Gebrauch an den Adapter angebracht werden können. Eine vorgefertigte und gebrauchsfertige Anordnung ist daher nicht möglich. Aus Gründen der Sterilität und der einfacheren Handhabung besteht allerdings ein Bedürfnis, eine gebrauchsfertige Transfervorrichtung mit Behälter(n) und Abgabevorrichtung in einer sterilen Verpackung bereitzustellen, sodass zur Benutzung nur wenige Handgriffe und insbesondere kein Anschluss von sterilitätssensiblen Komponenten erforderlich sind, um die medizinische(n) Substanz(en) in die Abgabevorrichtung zu transferieren.

Allgemein besteht zudem das Problem, dass Transfervorrichtungen mit einem Adapter bei Anordnung aller benötigten Komponenten (Transfervorrichtung, Behälter, Injektionsspritze) eine vergleichsweise grosse Baugrösse mit schlechter Platzausnutzung haben und für eine gebrauchsfertige Anordnung nur wenig geeignet sind. Aufgrund der ungünstigen Anordnung muss eine Verpackung zudem besonders stabil gewählt werden, damit keine Beschädigung infolge mechanischer Einwirkung auftritt. Ausserdem sind bekannte Transfervorrichtungen mit einem Adapter für einen Behälter für ein Fluid meist nur für eine konkrete Konfiguration vorgesehen und damit in der Anwendung beschränkt.

Es ist daher eine Aufgabe der Erfindung, eine Vorrichtung der eingangs genannten Art zu schaffen, bei der die erwähnten Nachteile vermieden werden und welche insbesondere eine kompakte Bauweise einer Transfervorrichtung erlaubt. Weiter soll die Vorrichtung die Möglichkeit für eine vielseitig anwendbare Transfervorrichtung bereitstellen. Die Vorrichtung soll ausserdem Transfervorrichtungen ermöglichen, welche zuverlässig funktionieren und auch von nicht besonders geschultem Personal leicht zu bedienen sind. Ausserdem soll sie einfach und preiswert herzustellen sein, da die Vorrichtung Bestandteil einer Einwegverpackung ist, die nach Gebrauch entsorgt werden muss.

Diese Aufgaben werden erfindungsgemäss mit einer Vorrichtung gelöst, welche die Merkmale im Anspruch 1 aufweist. Dieser betrifft einen Adapter für eine Transfervorrichtung für ein Fluid, zum Anschluss eines Behälters enthaltend das Fluid, wobei der Behälter mit einem penetrierbaren Verschlusselement verschlossen ist und der Adapter einen dichtend in einer Aufsatzrichtung auf eine mit dem Verschlusselement verschlossene Behältermündung aufsetzbaren Aufsatz aufweist, an welchem ein Penetrationselement derart gelagert ist, dass das Penetrationselement mit Hilfe von Führungsmitteln zwischen einer Ruhelage und einer Entnahmelage verschiebbar ist, wobei das Penetrationselement eine Hohlnadel aufweist, mit welcher in der Entnahmelage das Verschlusselement penetrierbar ist, wobei der Adapter einen Anschlussstutzen mit einer Anschlusskupplung zum fluiddichten Anschluss eines weiteren Adapters aufweist und ein Transferkanal von einer Mündung am Anschlussstutzen bis zu einer Mündung an der Hohlnadel verläuft, sodass in der Entnahmelage des Penetrationselements die Mündung des Transferkanals am Anschlussstutzen mit einem Innenraum eines angeschlossenen Behälters kommuniziert. Die Erfindung zeichnet sich dadurch aus, dass der Anschlussstutzen quer, insbesondere weitgehend senkrecht, zur Aufsatzrichtung, vorzugsweise am Penetrationselement, angeordnet ist. Vorzugsweise ist der Anschlussstutzen mit seiner Längsachse senkrecht zur Aufsatzrichtung angeordnet.

Der erfindungsgemässe Adapter erlaubt ein Aneinanderkoppeln von mehreren Adaptern, um eine Transfervorrichtung zu bilden. Aufgrund der so erreichten Modularität sind damit vielseitige Konfigurationen einer Transfervorrichtung durch Zusammenschluss von mehreren Adaptern möglicht. Zudem erlaubt der Adapter aufgrund der Ruhelage des Penetrationselements eine gebrauchsfertige Anordnung eines Behälters am Adapter bzw. mehrerer Behälter in einer Transfervorrichtung mit mehreren Adaptern, welche erst kurz vor Gebrauch auf einfache Weise durch Eindrücken des Penetrationselements sicher aktiviert werden können, ohne dass die Gefahr einer Kontamination besteht. Durch den quer, insbesondere senkrecht, zur Aufsatzrichtung angeordneten Anschlussstutzen kann zudem eine kompakte Baugrösse erreicht werden, womit die gebrauchsfertige Anordnung auch z.B. für Lagerung und Versand besonders geeignet ist.

Die Aufsatzrichtung des Adapters auf den Behälter entspricht dabei in der Regel einer Längsachse des beispielsweise als Glasfiole ausgebildeten Behälters. Auf diese Weise können mehrere Adapter mit jeweils einem in Aufsatzrichtung angeschlossenen Behälter z.B. parallel in entweder gleichsinniger Ausrichtung oder in, bezüglich einer Achse der Anschlussstutzen um 180 Grad gedrehten, entgegen gesetzten (antiparallelen) Ausrichtung angeordnet sein. Letztere Anordnung ist insbesondere für den Fall vorteilhaft, dass einer der Behälter ein Medikament in flüssiger Form und der andere Behälter ein Medikament in fester Form umfasst, welches vor der Verabreichung im flüssigen Medikament gelöst werden soll. Eine gleichgerichtete (parallele) Anordnung ist insbesondere für den Fall von zwei Flüssigkeiten vorteilhaft, welche direkt vor der Anwendung zu mischen sind.

Damit können mit demselben erfindungsgemässen Adapter verschiedene Transfervorrichtungen realisiert werden, was eine besonders vielseitige Anwendung erlaubt. Insbesondere kann aufgrund der Anordnung des Anschlussstutzens in Querrichtung eine Gesamtbaugrösse der Transfervorrichtung wesentlich verkleinert werden, da die Adapter den Anforderungen entsprechend sowie den Dimensionen von z.B. Behältern und Abgabevorrichtung angepasst z.B. nebeneinander oder gegeneinander versetzt angeordnet sein können. Der Anschlussstutzen kann hierzu je nach Anforderung unterschiedliche Längen aufweisen, welche an die bekannten Dimensionen bekannter Behälter angepasst sind, sodass eine möglichst dichte Packung mehrer Behälter möglich ist.

Der Aufsatz des Adapters kann dabei vorteilhaft einen Zentrierring tragen, der die Hohlnadel zusätzlich führt und zentriert. Das Durchstechen des Verschlusselements erfolgt so an einer genau definierten Stelle. Ausserdem werden unerwünschte Querbewegungen durch fehlerhafte Handhabung beim Einstechen vermieden. In der Entnahmelage ist das Penetrationselement damit auch stabilisiert, sodass Kräfte auf einen am Penetrationselement ausgebildeten Anschlussstutzen über den Zentrierring zum Teil vom Aufsatz aufgenommen werden können.

Zur weiteren Stabilisierung kann das Penetrationselement ausserdem Rastmittel aufweisen, mit denen es in der Entnahmelage verrastbar ist. Dadurch ist gewährleistet, dass bei der Entnahme des Fluids bzw. bei Betätigung des Penetrationselements eines angeschlossenen weiteren Adapters keine unerwünschten Relativbewegungen auftreten.

Weitere Vorteile können erreicht werden, wenn die Führungsmittel wenigstens einen Wandabschnitt aufweisen, der das Penetrationselement mit dem Aufsatz verbindet und dieses in der Ruhelage hält, wobei der Wandabschnitt derart verformbar ist, dass er das Penetrationselement bis zum Erreichen der Entnahmelage führt. Durch die feste Verbindung zwischen Aufsatz und Penetrationselement ist einerseits eine Verdrehsicherung gewährleistet, anderseits in Folge der Verformbarkeit aber auch eine gute Geradeführung.

Der verformbare Wandabschnitt kann beispielsweise wenigstens eine das Penetrationselement umgebende, federelastische Membran sein. Diese kann derart verformbar sein, dass sie das Penetrationselement in der Entnahmelage unter der Federvorspannung fixiert. Die kuppelartig ausgebildete Membran ist somit in Folge ihrer Federeigenschaften sowohl in der Ruhelage als auch in der Entnahmelage vorgespannt. Dabei wird der "Knackfrosch"-Effekt der Membran ausgenutzt. Selbstverständlich können zum Fixieren des Penetrationselements in der Entnahmelage aber auch Rastmittel angeordnet sein, so dass die Federvorspannung der Membran für das Halten der Entnahmelage unerheblich ist.

Die Membran kann ganz unterschiedlich ausgebildet sein und beispielsweise auch in Umfangsrichtung aus mehreren, von einander abgetrennten Segmenten bestehen. Über die Zwischenräume zwischen den Segmenten kann in der Entnahmelage Atmosphärenluft nachströmen. Es ist aber auch denkbar, dass eine in der Ruhelage geschlossene Membran mit Sollbruchstellen versehen ist, welche beim Überführen des Penetrationselements in die Entnahmelage aufplatzen. Derartige Sollbruchstellen haben den Vorteil, dass die Membran in der Ruhelage relativ formsteif ist und erst nach dem Aufplatzen der Sollreisslinien eine erhöhte Elastizität aufweist. Ausserdem kann auch hier in der Entnahmelage über die aufgeplatzten Sollreisslinien Luft aus der Atmosphäre nachströmen.

Alternativ kann das Führungsmittel aber auch wenigstens zwei Stege aufweisen, welche die genannten Wandabschnitte bilden und von denen jeder derart mit wenigstens einem Biegegelenk versehen ist, dass die Stege bei einer Verschiebung des Penetrationselements in die Entnahmelage zusammenfaltbar sind. Die Stege bilden auf diese Weise spinnenartige Beine des Penetrationselements, die sich im Verlauf der Linearbewegung vorzugsweise nach aussen falten lassen.

Alternative Ausgestaltungen der Führungsmittel wären selbstverständlich denkbar. So könnte der Wandabschnitt beispielsweise auch als Faltenbalg ausgebildet werden, der sich zieharmonikaartig zusammenpressen lässt. Denkbar wäre auch eine Anzahl miteinander verbundene Ringe oder Ringsegmente, die sich teleskopartig zusammenschieben lassen.

Ein wesentlicher Vorteil wird in jedem Fall erreicht, wenn die Führungsmittel einstückig mit dem Aufsatz und/oder mit dem Penetrationselement verbunden sind. Die gesamte Struktur lässt sich auf diese Weise als Spritzgussteil in Kunststoffmaterial einstückig herstellen. Insbesondere ist es auch vorteilhaft, den Anschlussstutzen einstückig mit dem Penetrationselement zu fertigen.

In bestimmten Fällen kann es allerdings fabrikationstechnisch auch zweckmässig sein, einzelne Bauteile separat herzustellen und anschliessend miteinander zu verschweissen oder auf andere Weise miteinander zu verbinden. Bevorzugt sind in diesem Fall beispielsweise die Führungsmittel über eine Schnappkupplung mit dem Aufsatz verbunden. Damit können auf einfache Weise unterschiedlich ausgebildete Führungsmittel oder Penetrationselemente mit angeformten Führungsmitteln über eine einheitliche Kupplung auf identisch ausgebildete Aufsätze aufgeschnappt werden. Aufgrund des so erreichten modularen Aufbaus des Adapters selbst wird durch Nutzung gleichartiger Teile für unterschiedliche Ausführungen die Wirtschaftlichkeit des Adapters weiter verbessert. Zudem können unterschiedliche Materialeigenschaften optimal miteinander gepaart werden. Optimal sind dabei beispielsweise Werkstoffe, welche mit einer Vielzahl von Verfahren sterilisierbar sind (z. B. strahlensterilisierbar, dampfsterilisierbar), ohne dabei Schaden zu erleiden.

Eine weitere sehr vorteilhafte Ausführung besteht in der Wahl von möglichst transparenten Kunststoffen für die einzelnen Bauteile des Adapters. Dadurch kann das fertig abgefüllte und montierte Produkt zerstörungsfrei visuell auf jegliche Defekte inspiziert werden.

Besonders vorteilhaft ist eine Ausführung des Adapaters mit einem Belüftungskanal zum Belüften des Behälters bei einer Entnahme des Fluids. Der wenigstens eine, vorzugsweise entlang der Hohlnadel verlaufende, Belüftungskanal bewirkt, dass das Innere des Behälters in der Entnahmelage mit der Atmosphäre kurzgeschlossen wird. Der Aufbau eines stetig steigenden Unterdrucks im Behälter wird damit vermieden, da jeweils so viel Luft in den Behälter nachströmt, wie Flüssigkeit entfernt wird. Damit kann unter Aufrechterhalten von sterilen Bedingungen ein Rückströmen von Flüssigkeit in einen an den Adapter angeschlossene Behälter vermieden werden, wenn über den Transferkanal Flüssigkeit abgeführt wird.

Selbstverständlich ist es denkbar, mehrere Lüftungskanäle anzuordnen, welche entlang der Hohlnadel entweder linear oder auch schraubenlinienförmig verlaufen können. Denkbar wäre auch die Anordnung eines Belüftungskanals an einem von der Hohlnadel getrennten Element, beispielsweise in der Form einer separaten Belüftungsnadel.

Je nach Erfordernis kann in einer bevorzugten Ausführungsform der Belüftungskanal als innenliegender Fluidkanal der Hohlnadel ausgebildet sein. Auf diese Weise kann ausgeschlossen werden, dass der Belüftungskanal ungewollt von dem penetrierbaren Verschlusselement verschlossen werden kann.

Mit Vorteil ist jedoch der Belüftungskanal als Nut auf der Aussenseite der Hohlnadel ausgebildet. Vorzugsweise sind es mehrere parallele Nuten. Ein derartiges Bauteil ist einfach herzustellen und bei ausreichender Nuttiefe wird selbst dann ein genügend grosser Durchtrittsquerschnitt freigehalten, wenn das Verschlusselement aus sehr weichem und elastischem Material besteht.

Trotz des Belüftungskanals ist eine schmutzfreie und sogar eine keimfreie Umgebung im Behälter gewährleistet, wenn gemäss einer weiteren Ausgestaltung die zugeführte Luft ein Filter passieren muss. Je nach Beschaffenheit dieses Filters können auch kleinste Partikel oder Lebewesen zurückgehalten werden. Bevorzugt umfasst der Adapter daher wenigstens ein Filter, über das in der Entnahmelage Luft aus der Atmosphäre über den Belüftungskanal in den Behälter zuführbar ist.

Das Filter kann grundsätzlich am Penetrationselement angeordnet sein und sich zusammen mit diesem verschieben, oder es kann starr am Aufsatz gehalten sein. Wichtig ist in jedem Fall, dass die dem Belüftungskanal zugewandte Seite des Filters Bestandteil eines gegenüber der Atmosphäre steril abgegrenzten Raumes ist. Das Filter kann beispielsweise am Penetrationselement vorzugsweise am atmosphärenseitigen Ende des Belüftungskanals angeordnet sein, und zwar beispielsweise an einem die Hohlnadel umgebenden Kragen, der mit Öffnungen versehen ist und der gleichzeitig das Filter abstützt bzw. trägt. Diese nahe am Belüftungskanal angeordnete Variante hat den Vorteil, dass das Filter flächenmässig relativ klein gehalten werden kann. Das Filter kann alternativ aber auch in einem separaten Filtereinsatz angeordnet sein, welcher z.B. in einen dafür vorgesehenen, mit dem Belüftungskanal kommunizierenden Aufnahmeraum am Adapter eingesetzt werden kann. Beispielsweise kann es vorteilhaft sein, den Aufnahmeraum für den Filtereinsatz bei einer Ansaugöffnung für die nachströmende Luft anzuordnen.

Bei einer Zuordnung des Filters am Aufsatz erfolgt die Anordnung vorzugsweise in einem der Behältermündung zugewandten Bereich. Das Filter kann dabei in einer Halterung angeordnet sein, welche das Penetrationselement kreisringförmig umgibt, wenn das Penetrationselement in der Entnahmelage ist. Diese Anordnung hat den Vorteil, dass der in der Ruhelage steril zu haltende Raum sehr klein gehalten werden kann.

Eine Ausführungsform des Adapters, die nicht Teil der Erfindung ist, kann ein Kopplungsmittel zum Ankoppeln einer Abgabevorrichtung in einer Kopplungsrichtung umfassen. Abgabevorrichtungen weisen typischerweise eine längliche Bauweise auf wie beispielsweise Spritzen bekannter Bauart. Die Kopplungsrichtung, in welcher die Abgabevorrichtung an das Kopplungsmittel gekoppelt wird, entspricht dabei der Längsrichtung der Abgabevorrichtung. Der Adapter umfasst dabei einen als Entnahmekanal ausgebildeten, weiteren Fluidkanal, welcher von einer Mündung am Kopplungsmittel bis zu einer Mündung an der Hohlnadel verläuft. Der Entnahmekanal verläuft dabei derart, dass in der Entnahmelage des Penetrationselements die Mündung des Entnahmekanals beim Kopplungsmittel mit dem Innenraum eines angeschlossenen Behälters kommuniziert. Mit Vorteil sind dabei die durch das Kopplungsmittel vorgegebene Kopplungsrichtung sowie die Aufsatzrichtung des Behälters parallel zueinander ausgerichtet. Das Kopplungsmittel kann dabei bekannte Kupplungen umfassen, bevorzugt einen Innenkonus einer Luer-Kupplung.

Das Kopplungsmittel kann dabei derart bezüglich der Kopplungsrichtung seitlich versetzt am Adapter ausgebildet sein, dass eine an der Abgabevorrichtung angebrachte Nadel oder Kanüle sich parallel zum Behälter längs diesem erstrecken kann. Hierzu kann eine Hülse zur Aufnahme der Kanüle oder Nadel direkt am Adapter, insbesondere am Penetrationselement ausgebildet sein. Die Zuführung des Fluids kann in diesem Fall über eine separate Füllöffnung der Abgabevorrichtung erfolgen z. B. analog der weiter unten beschriebenen Ausführungsform eines Kopplungsmittels mit Hahnsystem.

Typischerweise ist bei dieser Ausführungsform des Adapters kein Belüftungskanal erforderlich, da sie dafür vorgesehen ist, in der Transfervorrichtung über einen Unterdruck am Kopplungsmittel z.B. durch Aufziehen einer als Spritze ausgebildeten Abgabevorrichtung über den Transferkanal ein Fluid in den angeschlossenen Behälter anzusaugen. Im angeschlossenen Behälter kann sich das Fluid mit einem anderen dort vorhandenen Fluid mischen oder einen im Behälter vorhandenen Feststoff lösen. Über den Entnahmekanal kann dann das Gemisch bzw. die Lösung entnommen bzw. der Abgabevorrichtung zugeführt werden.

In einer weiteren alternativen Ausführungsform des Adapters ist ein zweiter Anschlussstutzen mit einer Anschlusskupplung zum fluiddichten Anschluss eines weiteren Adapters vorhanden. Der zweite Anschlussstutzen ist dabei bevorzugt quer, insbesondere senkrecht, zur Aufsatzrichtung angeordnet. Besonders bevorzugt liegen die Längsachsen der beiden Anschlussstutzen in derselben, quer zur Aufsatzrichtung angeordneten Ebene. Der Adapter umfasst dabei einen weiteren, als zweiten Transferkanal ausgebildeten Fluidkanal, welcher von einer Mündung am zweiten Anschlussstutzen bis zu einer Mündung an der Hohlnadel verläuft, sodass die Mündung des zweiten Transferkanals am zweiten Anschlussstutzen mit dem Innenraum des angeschlossenen Behälters kommuniziert. Diese Ausführungsform des Adapters eignet sich für den Anschluss zweier weiterer Adapter, wenn z.B. mehr als zwei Medikamente vor der Verabreichung gemischt werden müssen.

An sämtliche vorgenannten Ausführungsformen eines erfindungsgemässen Adapters kann in einer Transfervorrichtung aber auch ein Adapter angeschlossen werden, welcher nur für die Zufuhr eines Fluids zu der Abgabevorrichtung ausgebildet ist. Indem für den Anschluss der Abgabevorrichtung ein separater Adapter vorgesehen ist, kann dieser besonders gut an die spezifischen Erfordernisse angepasst sein.

Ein derartiger Adapter, welcher auch losgelöst von den vorgenannten Adaptern vorteilhaft ist, umfasst ein Kopplungsmittel zum Ankoppeln einer Abgabevorrichtung in einer Kopplungsrichtung, welche bevorzugt einer Längsrichtung der Abgabevorrichtung entspricht. Der Adapter umfasst einen Anschlussstutzen mit einer Anschlusskupplung für einen weiteren Adapter, wobei ein Fluidkanal durchgehend von einer Mündung am Anschlussstutzen zu einer Mündungsöffnung am Kopplungsmittel verläuft. Der Anschlussstutzen ist bevorzugt quer, insbesondere senkrecht, zur Kopplungsrichtung angeordnet und weist ein Gehäuse mit einem Aufnahmeraum für z. B. eine Nadel oder Kanüle der Abgabevorrichtung auf. Das Gehäuse wirkt dabei im Sinne von bekannten Schutzkappen für Injektionsnadeln.

Aufgrund der analog den vorgenannten Adaptern zum Anschluss eines Behälters gewählten Anordnung der Anschlussstutzen kann auch der nur für die Zufuhr eines Fluids zu einer Abgabevorrichtung vorgesehene Adapter auf vorteilhafte Weise vielseitig und platzsparend in einer Transfervorrichtung angeordnet werden. Bevorzugt ist dabei das Kopplungsmittel für die Abgabevorrichtung im Bereich einer nach aussen offenen Einführöffnung des Aufnahmeraums ausgebildet, wobei sich der Aufnahmeraum vorzugsweise weitgehend in Kopplungsrichtung erstreckt. Die Mündungsöffnung ist dabei derart angeordnet, dass eine Füllöffnung der Abgabevorrichtung bei angekoppelter Abgabevorrichtung fluiddicht an die Mündungsöffnung des Fluidkanals anschliessbar ist.

Insbesondere umfasst dabei das Kopplungsmittel einen Innenkonus, wobei die Mündungsöffnung auf eine Mantelfläche des Innenkonus angeordnet ist. Bei einer entsprechend ausgebildeten Abgabevorrichtung, d.h. bei einer Abgabevorrichtung mit entsprechendem Aussenkonus als komplementäres Kopplungsmittel, ist die Füllöffnung auf der Mantelfläche des Aussenkonus angeordnet. Die Füllöffnung ist dabei bevorzugt zusätzlich zu einer Abgabeöffnung vorhanden. Damit ist der Vorteil verbunden, dass beispielsweise bei in die Abgabeöffnung eingesetzter Kanüle oder Nadel die Abgabevorrichtung über die separate Füllöffnung befüllt werden kann.

Eine besonders für diese Ausführungsform des Adapters geeignete Abgabevorrichtung ist im Folgenden beschrieben. Insbesondere kann die nachfolgend beschrieben Abgabevorrichtung bereits mit dem Adapter versehen sein, womit z.B. eine Injektionsnadel im Aufnahmeraum geschützt ist und die Abgabevorrichtung über den Anschlussstutzen des Adapters befüllt werden kann.

Es versteht sich aber, dass auch andere Abgabevorrichtungen, welche über eine für die Abgabe eines Fluids vorgesehene Abgabeöffnung befüllt werden können, für diese Ausführungsform des Adapters geeignet sind. In diesem Fall ist die Mündung des Fluidkanals am Kopplungsmittel entsprechend der Lage der Abgabeöffnung der Abgabevorrichtung angeordnet.

Eine besonders bevorzugte Ausführungsform einer Abgabevorrichtung, welche auch losgelöst von den hier beschriebenen Adaptern oder derartige Adapter umfassenden Transfervorrichtungen vorteilhaft anwendbar ist, umfasst jedoch eine separate Füllöffnung. Eine separate Füllöffnung ist jedoch meist insofern problematisch bzw. aufwändig, als dass sie auch separat verschlossen werden muss, um eine Abgabe des Fluids durch eine Abgabeöffnung der Abgabevorrichtung sicherzustellen. Die folgend beschriebene Abgabevorrichtung löst dieses Problem auf konstruktiv einfache sowie sichere und einfach zu bedienende Weise.

Eine derartige Abgabevorrichtung für ein Fluid umfasst einen Grundkörper mit einem Innenraum zur Aufnahme des Fluids, wobei in einem Abgabebereich eine Abgabeöffnung ausgebildet ist, welche über einen Fluidkanal mit dem Innenraum kommuniziert und an welcher das Fluid bei Bedarf abgebbar ist. Im Abgabebereich ist ein Kopplungsmittel zum Ankoppeln der Abgabevorrichtung an eine Transfervorrichtung für eine Fluid ausgebildet, welches zum Ankoppeln in einer Kopplungsrichtung in ein entsprechend ausgebildetes Kopplungsmittel der Transfervorrichtung einbringbar ist. Das Kopplungsmittel der Abgabevorrichtung weist dabei eine in einer Füllstellung der Abgabevorrichtung mit dem Innenraum fluidschlüssig kommunizierende Füllöffnung auf, über welche die Abgabevorrichtung mit dem Fluid befüllbar ist. Das Kopplungsmittel ist dabei derart ausgebildet, dass der Fluidschluss zwischen Füllöffnung und Innenraum des Grundkörpers unterbrechbar ist. Die Abgabevorrichtung zeichnet sich dadurch aus, dass das Kopplungsmittel der Abgabevorrichtung um eine längs der Kopplungsrichtung ausgerichtete Drehachse rotierbar am Grundkörper gelagert ist und der Fluidschluss zwischen Füllöffnung und Innenraum aufgrund einer derartigen relativen Drehung zwischen Grundkörper und Kopplungsmittel unterbrechbar ist.

Eine derartige Abgabevorrichtung kann allgemein an eine mit einem entsprechenden Kopplungsmittel versehene Füll- oder Transfervorrichtung angeschlossen werden. Im Folgenden ist die Abgabevorrichtung allerdings, ohne Beschränkung der allgemeinen Anwendbarkeit, im Zusammenhang mit einem Adapter für eine Transfervorrichtung beschrieben.

Bevorzugt weist die Abgabevorrichtung fest am Grundkörper ausgebildete Eingriffsmittel auf, welche zur Sicherung der angekoppelten Abgabevorrichtung mit entsprechenden Eingriffsmitteln am Adapter, insbesondere bajonettartig, im Eingriff stehen.

Der bajonettartige Eingriff kann somit einfach gelöst werden, indem der Grundkörper der Abgabevorrichtung relativ zum Adapter um eine der Kopplungsrichtung entsprechende Achse gedreht wird.

Das Kopplungsmittel der Abgabevorrichtung weist bevorzugt Mittel zur verdrehsicheren Halterung im Kopplungsmittel des Adapters auf. Damit wird sichergestellt, dass bei angekoppelter Abgabevorrichtung bei einer Drehung des Grundkörpers gegenüber dem Adapter der Grundkörper auch gegenüber dem Kopplungsmittel der Abgabevorrichtung gedreht wird. Auf diese Weise ist aufgrund der Drehung des Grundkörpers bezüglich des Adapters zum einen der Fluidschluss zwischen Füllöffnung und Innenraum unterbrechbar. Zum anderen ist gegebenenfalls gleichzeitig der Eingriff der bajonettartigen Eingriffsmittel des Grundkörpers mit den Eingriffsmitteln des Adapters lösbar, sodass die Abgabevorrichtung entsichert und vom Adapter entkoppelbar ist. Die Mittel zur verdrehsicheren Halterung können dabei Nuten oder Rippen umfassen, welche in Kopplungsrichtung angeordnet sind, sodass das Kopplungsmittel in dieser Richtung in das Kopplungsmittel des Adapters eingebracht bzw. aus diesem ausgebracht werden kann. Bevorzugt ist die Füllöffnung bezüglich der Kopplungsrichtung seitlich am Kopplungsmittel der Adaptervorrichtung ausgebildet, sodass die Füllöffnung fluiddicht an eine entsprechend am Adapter ausgebildete Transferöffnung eines Fluidkanals angeschlossen werden kann.

Mit Vorteil ist das Kopplungsmittel der Abgabevorrichtung als Manschette ausgebildet, welche an einem in Kopplungsrichtung am Grundkörper angeordneten Aufsatzstutzen gelagert ist. Dabei ist die Füllöffnung mit Vorteil auf einer Mantelfläche der Manschette ausgebildet. Insbesondere ist die Füllöffnung dabei auf einem konisch ausgebildeten Abschnitt der Mantelfläche ausgebildet, um auf einfache Weise einen fluiddichten Ankopplung in einem entsprechend konischen Sitz des Kopplungsmittels des Adapters zu gewährleisten.

Vorzugsweise ist der Aufsatzstutzen dabei in Längsrichtung des Grundkörpers an diesem angeordnet, wobei die Abgabeöffnung stirnseitig an einem vom Grundkörper abgewandten Längsende des Aufsatzstutzens angeordnet ist und der Fluidkanal im Aufsatzstutzen von der Abgabeöffnung zum Innenraum verläuft.

Mit Vorteil ist der Fluidschluss zwischen Füllöffnung und Innenraum über jeweils eine Querbohrung im Kopplungsmittel und im Aufsatzstutzen hergestellt, welche derart angeordnet und ausgebildet sind, dass sie in der Füllstellung der Abgabevorrichtung miteinander fluchten. Die Querbohrung im Aufsatzstutzen kommuniziert dabei mit dem Fluidkanal der Abgabevorrichtung. Querbohrung bezeichnet hier und im Folgenden eine Bohrung quer zur Kopplungsrichtung bzw. Rotationsachse des Kopplungsmittels. Der innen liegende Fluidkanal des Anschlusszapfens mündet an einem vom Aufnahmeraum abgewandten Längsende in einer zur Abgabe des Fluids aus dem Aufnahmeraum vorgesehenen Abgabeöffnung. Damit ist die Füllöffnung unabhängig von der Abgabeöffnung in einem seitlichen Bereich angeordnet, womit die Abgabevorrichtung beispielsweise bei aufgesetzter Nadel oder Kanüle oder bei verschlossener Abgabeöffnung befüllt werden kann. Damit erschliesst sich unmittelbar, dass der hygienisch relevanteste Bereich, nämlich der Abgabebereich oder eine Injektionsnadel oder Kanüle, von einem Benutzer nicht direkt manipuliert werden muss, um die Füllöffnung zu schliessen.

Um eine nur einmalige Verwendbarkeit der Abgabevorrichtung sicherzustellen, sind vorzugsweise Sicherungsmittel vorhanden, welche eine Drehung des Grundkörpers bezüglich dem Kopplungsmittel der Abgabevorrichtung nur in einer Entkopplungsrichtung, insbesondere nur in einem beschränkten Bereich, zulassen. Bevorzugt sind hierzu Rastzungen ausgebildet, über welche eine Drehung entgegen der Entkopplungsrichtung rätschenartig blockierbar ist. Ebenso können mit Vorteil Anschläge vorgesehen sein, welche eine Drehbarkeit auf einen gewissen Winkelbereich einschränken. Bevorzugt sind die Sicherungsmittel am Kopplungsmittel der Abgabevorrichtung ausgebildet und wirken mit grundkörperfesten Elementen zusammen, insbesondere beispielsweise mit den vorgenannten Eingriffsmitteln des Grundkörpers.

Es erschliesst sich unmittelbar, dass eine mit einem derartigen rotierbar am Grundkörper gelagerten Kopplungsmittel versehene Abgabevorrichtung vielseitig anwendbar ist und deutlich Vorteile hinsichtlich Sterilitätsanforderungen, Konstruktion und Handhabung hat. Insbesondere kann auch eine korrekte und nur einmalige Benutzung der Abgabevorrichtung sichergestellt sein.

Die Abgabevorrichtung kann dabei mit Vorteil bei entsprechend ausgebildeten oben genannten Adaptern oder einer Transfervorrichtung umfassend derartige Adapter (siehe unten) zur Anwendung kommen. Insbesondere kann bei allen hier beschriebenen Adaptern, welche Kopplungsmittel zur Ankopplung einer Abgabevorrichtung aufweisen, ein entsprechend ausgebildetes Kopplungsmittel mit einer Öffnung zur Fluidzuführung zur Füllöffnung der Abgabevorrichtung vorgesehen sein, sodass die Abgabevorrichtung entsprechend angekoppelt und befüllt werden kann. Insbesondere kann die Abgabevorrichtung in einer hier beschriebenen Transfervorrichtung in einem entsprechend ausgebildeten Adapter angeordnet sein.

Ein weiterer Aspekt der Erfindung umfasst eine Anordnung aus wenigstens zwei Adaptern, welche eine Transfervorrichtung, insbesondere eine modulare Transfervorrichtung, für den Transfer eines Fluids aus einem anschliessbaren Behälter in eine ankoppelbare Abgabevorrichtung bereitstellt, insbesondere in eine vorgängig beschriebene Abgabevorrichtung mit separater Füllöffnung.

Die Transfervorrichtung umfasst wenigstens einen der vorgenannten Adapter mit einem Belüftungskanal und einen zweiten der vorgenannten Adapter mit einem Kopplungsmittel zum Ankoppeln einer Abgabevorrichtung in einer Kopplungsrichtung.

Die wenigstens zwei Adapter sind in der Transfervorrichtung über die Anschlusskupplungen der Anschlussstutzen derart direkt oder indirekt aneinander angeschlossen bzw. miteinander verbunden, dass bei angeschlossenem Behälter bzw. angeschlossenen Behältern vom Kopplungsmittel des zweiten Adapters bis zum Belüftungskanal des ersten Adapters gesamthaft ein fluiddichter Kanal bereitgestellt ist. Wird in der Abgabevorrichtung ein Unterdruck erzeugt, beispielsweise beim Aufziehen einer angekoppelten Spritze, so kann das Fluid aus dem Behälter am ersten Adapter über den zweiten Adapter in die Abgabevorrichtung strömen. Der Belüftungskanal des ersten Adapters ermöglicht dabei ein Nachströmen von, gegebenenfalls keimfrei gefilterter, Luft aus der Atmosphäre.

Je nach Ausbildung des zweiten Adapters kann das Fluid dabei im zweiten Adapter direkt über den Fluidkanal dem Kopplungsmittel bzw. der Abgabevorrichtung zugeführt werden oder indirekt über einen am zweiten Adapter angeschlossenen Behälter. In letzterem Fall kann im angeschlossenen Behälter ein weiteres Medikament vorhanden sein, welches z.B. mit dem ersten Fluid mischbar (zweites Fluid) oder in diesem lösbar (Feststoff) ist.

Mit Vorteil sind dabei die Anschlusskupplungen der beiden Adapter derart komplementär zueinander ausgebildet sind, dass der zweite Adapter an den ersten Adapter direkt fluiddicht anschliessbar ist.

Alternativ umfasst die Transfervorrichtung mit Vorteil ein Kopplungsteil derart, dass der zweite Adapter an den ersten Adapter indirekt fluiddicht über das Kopplungsteil anschliessbar ist. Auf diese Weise brauchen in der Transfervorrichtung keine unterschiedlichen Adapter für unterschiedliche Anschlusskonfigurationen vorgesehen zu werden. Das Kopplungsteil kann jeweils derart angepasst sein, dass jeder Adapter mit jedem anderen Adapter verbunden werden kann. Dies hat den Vorteil, dass hinsichtlich der Anschlusskopplungen gleichartig ausgebildete Adapter zur Anwendung kommen können.

Mit besonderem Vorteil sind die Anschlusskupplungen der beiden Adapter derart ausgebildet, dass die Adapter in genau zwei relativen Ausrichtungen aneinander koppelbar sind, wobei bevorzugt die Anschlussrichtung des ersten Adapters und die Kopplungsrichtung des zweiten Adapters in einer der beiden Ausrichtungen entgegengesetzt und in der anderen Ausrichtung gleichgerichtet sind. Mit Vorteil sind hierzu an den Anschlusskopplungen zueinander komplementäre Rastmittel ausgebildet, welche insbesondere zum einen die Ausrichtungen der beiden Adapter vorgeben und vorzugsweise zum anderen die von den beiden Anschlusskopplungen hergestellte Verbindung der beiden Adapter sichern.

Damit ist, wie eingangs bereits erwähnt, der Vorteil verbunden, dass in der Transfervorrichtung verschiedene relative Anordnungen der Adapter, und damit der angeschlossenen Behälter, ermöglicht werden. Je nachdem ob zwei flüssige Medikamente zur Mischung vorgesehen sind (bevorzugt parallele Anordnung) oder ein festes Medikament in einem flüssigen Medikament/Lösungsmittel gelöst werden soll (bevorzugt antiparallele Anordnung), kann die Transfervorrichtung durch entsprechende relative Anordnung der Adapter in der Transfervorrichtung bzw. deren Anordnung in Bezug auf eine angekoppelte Abgabevorrichtung optimal an die Erfordernisse angepasst werden.

Insbesondere kann aber auch eine Gesamtbaugrösse der Transfervorrichtung wesentlich verkleinert werden, da die Adapter den Anforderungen entsprechend sowie den Dimensionen von z.B. Behältern und Abgabevorrichtung angepasst angeordnet werden können. Indem zwei Ausrichtungen vorgegeben sind, in welchen die relevanten Längsrichtungen (Aufsatzrichtung, Kopplungsrichtung) parallel bzw. antiparallel angeordnet sind, kann eine besonders kompakte Bauweise erreicht werden.

Es versteht sich, dass im Falle eines indirekten Anschlusses zweier Adapter über ein Kopplungsteil eine entsprechende Ausrichtung z.B. über das Kopplungsteil vorgegeben sein kann.

Mit besonderem Vorteil umfasst eine der Anschlusskupplungen der beiden Adapter einen Aussenkonus und die andere der beiden Anschlusskopplungen einen Innenkonus, wobei insbesondere der Innenkonus an der Anschlusskopplung des ersten, belüfteten Adapters ausgebildet ist. Damit lässt sich auf einfache Weise ein Zusammenschluss zweier Adapter mit guter Fluiddichtigkeit herstellen.

Im Fall eines indirekten Anschlusses über ein Kopplungsteil können beide Anschlusskopplungen denselben Konus aufweisen, wobei das Kopplungsteil entsprechend komplementäre Koni aufweist.

Die Transfervorrichtung kann neben dem ersten und dem zweiten Adapter auch noch wenigstens einen weiteren Adapter umfassen, welcher wie oben beschrieben mit zwei Anschlussstutzen versehen ist. Dabei sind die Anschlusskupplung des ersten Anschlussstutzens bevorzugt komplementär zur Anschlusskupplung des ersten Adapters und vorzugsweise die Anschlusskupplung des zweiten Anschlussstutzens komplementär zur Anschlusskupplung des zweiten Adapters ausgebildet, sodass der weitere Adapter gleichzeitig fluiddicht direkt an den ersten und den zweiten Adapter anschliessbar ist. Der weitere Adapter ist in diesem Fall gewissermassen fluidmässig zwischen den ersten und den zweiten Adapter zwischengeschaltet.

Es versteht sich, dass, je nach Anforderung, auch der wenigstens eine weitere Adapter mit Vorteil indirekt über ein Kopplungsteil an den ersten und/oder den zweiten Adapter angeschlossen sein kann.

Mögliche Anordnungen der Adapter umfassen beispielsweise, fluidmässig in der genannten Reihenfolge zusammengeschlossen: einen Adapter mit Belüftungskanal, einen Adapter mit zwei Anschlussstutzen und einen Adapter, welcher nur für die Zufuhr eines Fluids zu der Abgabevorrichtung ausgebildet ist. Dabei sind die Anschlussstutzen mit ihren Längsachsen bevorzugt in einer gemeinsamen Ebene angeordnet, wobei die Anschlussstutzen des Adapters mit zwei Anschlussstutzen derart unter einem Winkel zueinander angeordnet sein können, dass sich eine Dreiecksanordnung der drei Adapter nebeneinander ergibt.

Zum Schutz der Transfervorrichtung z.B. gegen mechanische Beschädigung beim Transport kann ein gemeinsames Gehäuse zum Schutz vorhanden sein, in welchem die Adapter der Transfervorrichtung angeordnet sind. Das Gehäuse kann dabei derart ausgebildet sein, dass auch eine angekoppelte Abgabevorrichtung darin aufgenommen sein kann. Dabei sind die Adapter in einer für die Benutzung vorgesehenen Konfiguration im Gehäuse angeordnet und können in den relativen Positionen gesichert sein. Das Gehäuse kann dabei Zugangasöffnungen aufweisen, durch welche die Penetrationselemente der einzelnen Adapter in die Entnahmelage gebracht werden können und/oder eine angekoppelte Abgabevorrichtung betätigt werden kann.

Insbesondere kann das Gehäuse auch Sicherungsmittel aufweisen, welche eine korrekte Handhabung der Transfervorrichtung vorgeben. Beispielsweise können die Sicherungsmittel derart ausgebildet sein, dass eine korrekte Reihenfolge der Betätigungen vorgegeben ist. Die Sicherungsmittel können z.B. derart ausgebildet sein, dass im Fall zweier Medikamentenbehälter ein Aufziehen der Abgabevorrichtung erst erfolgen kann, wenn die Penetrationselement beider Adapter in der Entnahmelage sind. Zudem können auch Sicherungsmittel vorhanden sein, welche eine nur einmalige Benutzung der Transfervorrichtung sicherstellen. Die Sicherungsmittel können aber auch entsprechende auf das Gehäuse aufgedruckte oder geprägte Hinweise umfassen.

Weitere Vorteile und Einzelmerkmale der Erfindung sind in den Zeichnungen dargestellt und werden nachstehend beschrieben. Es zeigen schematisch:
- Figur 1: Querschnittsansicht durch einen Adapter mit Belüftungskanal;
- Figur 2: Draufsicht in Richtung des Achsstutzens auf den Adapter gemäss Fig. 1;
- Figur 3: Draufsicht von einer Anschlussseite her auf den Adapter gemäss Fig. 1;
- Figur 4: Detailansicht der Hohlnadel mit Belüftungskanal des Adapters gemäss Fig. 1;
- Figur 5: äussere Schrägansicht auf den Adapter gemäss Fig. 1;
- Figur 6: Querschnittsansicht einer weiteren Ausführungsform eines Adapters mit Kopplungsmittel für eine Abgabevorrichtung;
- Figur 7: Draufsicht in Richtung des Achsstutzens auf den Adapter gemäss Fig. 6;
- Figur 8: Draufsicht auf eine Anschlussseite her auf den Adapter gemäss Fig. 6;
- Figur 9: äussere Schrägansicht auf den Adapter gemäss Fig. 6;
- Figur 10: Transfervorrichtung mit angekoppelter Abgabevorrichtung und angeschlossenen Behältern, wobei die Behälter antiparallel angeordnet sind;
- Figur 11: Transfervorrichtung mit Adapteranordnung zum parallelen Anschluss von zwei Behältern (Adapter ohne Aufsätze dargestellt);
- Figur 12: eine weitere Ausführungsform eines Adapters als Zwischenstück, mit zwei Anschlussstutzen (ohne Aufsatz dargestellt);
- Figur 13: Aufsatz für einen Adapter in einem Längsquerschnitt;
- Figur 14: Draufsicht in Längsrichtung von einer Kopplungsseite her auf den Aufsatz der Fig. 13;
- Figur 15: ein nicht erfindungsgemäßen Adapter zum Ankoppeln einer Abgabevorrichtung mit einer Hülse zur Aufnahme einer Injektionsnadel in einem Längsquerschnitt;
- Figur 16: Draufsicht in Längsrichtung auf eine Kopplungsseite des Adapters gemäss Fig. 15;
- Figur 17: Ausschnittsansicht gemäss Fig. 15 mit angekoppelter Abgabevorrichtung;
- Figur 18: Ausschnittsansicht gemäss Fig. 17 in einer zur Ansicht der Fig. 17 senkrechten Schnittebene;
- Figur 19: Draufsicht auf eine Manschette der Abgabevorrichtung gemäss Fig. 17 in Längsrichtung;
- Figur 20: seitliche Draufsicht auf die Manschette gemäss Fig. 19;
- Figur 21: Draufsicht auf einen Grundkörper der Abgabevorrichtung gemäss Fig. 17 ohne Manschette;
- Figur 22: Ausschnittsansicht eines Längsschnittes des Grundkörpers gemäss Fig. 21;
- Figur 23: Ausschnittsansicht gemäss Fig. 22 in einer zur Ansicht der Fig. 22 senkrechten Schnittebene;
- Figur 24: äussere Schrägansicht einer weiteren Ausführungsform eines erfindungsgemässen Adapters;
- Figur 25: den Adapter der Fig. 24 in einer weiteren äusseren Schrägansicht;
- Figur 26: Detailansicht einer Hohlnadel mit zwei innenliegenden Kanälen des Adapters gemäss Fig. 24;
- Figur 27: seitliche Draufsicht auf den Adapter gemäss Fig. 24;
- Figur 28a: Längsquerschnitt durch den Adapter der Fig. 24;
- Figur 28b: Längsquerschnitt durch den Adapter der Fig. 24 mit alternativer Fluidkanalführung;
- Figur 28c: Längsquerschnitt durch eine weitere Ausführungsform eine erfindungsgemässen Adapters;
- Figur 29: Draufsicht in Längsrichtung von einer Kopplungsseite her auf eine weitere Ausführungsform eines Aufsatzes für den Adapter der Fig. 24;
- Figur 30: Aufsatz gemäss Fig. 29 in einem Längsquerschnitt;
- Figur 31: Aufsatz gemäss Fig. 29 in einem weiteren Längsquerschnitt;
- Figur 32a: Filtereinsatz für einen erfindungsgemässen Adapter;
- Figur 32b: Schnittansicht des Filtereinsatzes gemäss Fig. 32a;
- Figur 33: Kopplungsteil zum Anschluss zweier erfindungsgemässer Adapter.

Im Folgenden sind einander entsprechende Bauteile mit gleichen Bezugszeichen versehen.

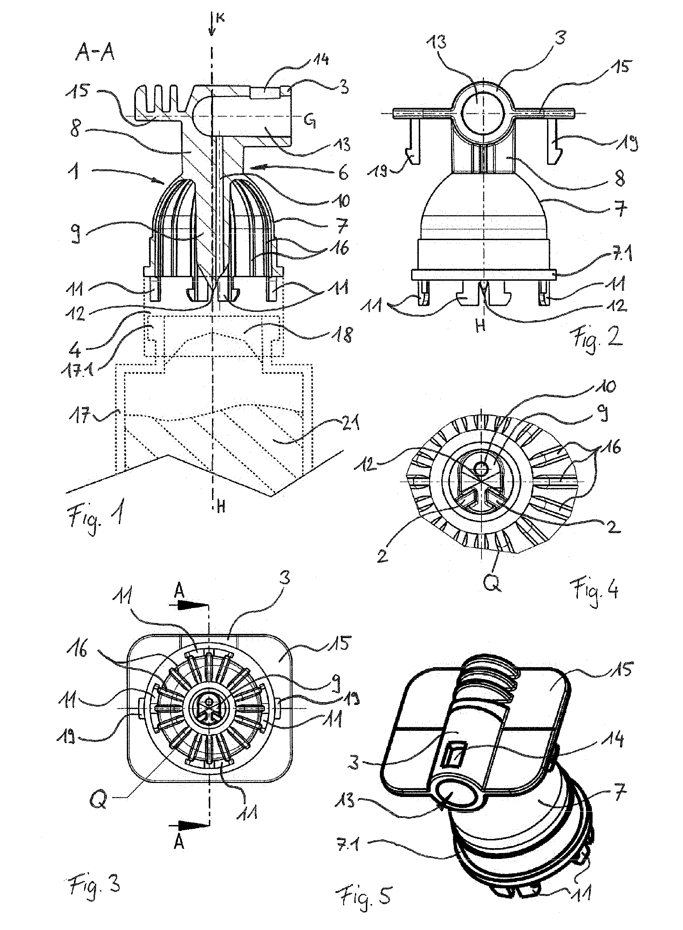

Figur 1 zeigt eine Schnittansicht in einer Ebene A gemäss Fig. 3 durch einen erfindungsgemässen Adapter 1 mit Belüftungskanal 2 (siehe Fig. 3 und 4). Figur 2 zeigt eine Frontalansicht und Fig. 3 und 4 eine Ansicht von einer zum Aufsatz auf einen Behälter 17 vorgesehenen Seite her, allerdings ohne einen vorliegend als separates Bauteil ausgebildeten Aufsatz 4 des Adapters 1. Figur 5 zeigt eine äussere Schrägansicht des Adapters 1. Im Folgenden sind die Fig. 1 bis 5 gemeinsam beschrieben. Die Schnittebene A ist senkrecht zu einer Achse G eines Anschlussstutzens 3 angeordnet und umfasst eine Gerade H, welche eine Aufsatzrichtung eines Behälters 17 bezeichnet. Die Aufsatzrichtung definiert sich dabei über eine Richtung, in welcher ein Behälter 17 auf den Adapter 1 aufgesetzt wird und entspricht in der Regel einer Längsrichtung sowohl des Behälters 17 als auch des Adapters 1.

Der Adapter 1 umfasst den Aufsatz 4 (gestrichelt) aus Kunststoffmaterial, der mit seiner Unterseite dichtend auf dem äusseren Randbereich des Behälters 17 aufliegt. Der Behälter 17 ist von einem penetrierbaren Verschlusselement 18 verschlossen und in Fig. 1 ebenfalls schematisch angedeutet (gestrichelt).

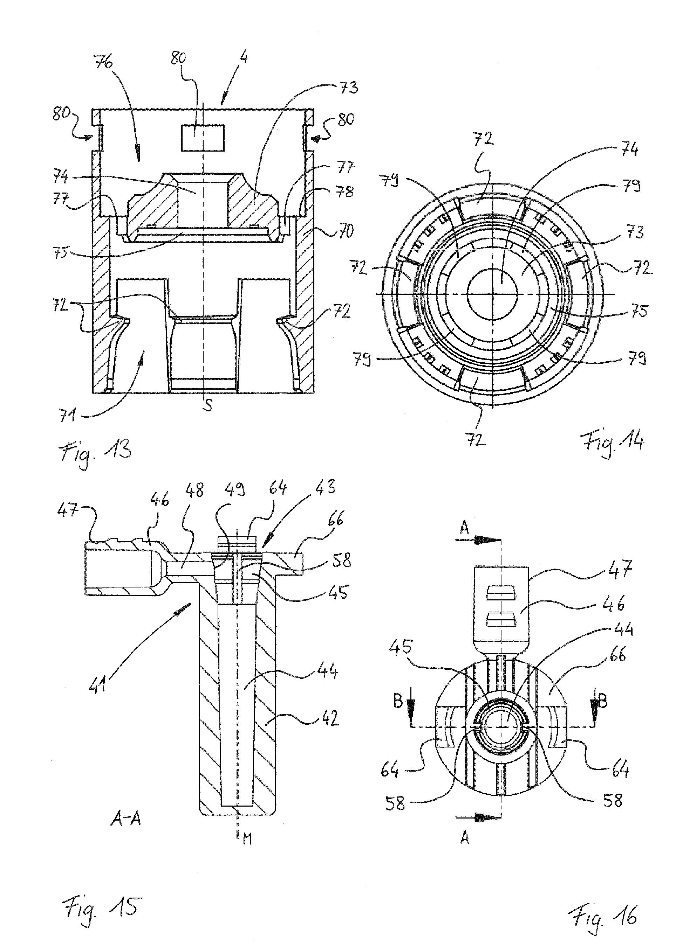

Der Adapter 1 umfasst weiter ein Penetrationselement 6 mit einem zentralen Grundkörper 8. Der Grundkörper 8 kann über eine kuppelartig gewölbte Membran 7 mit dem Aufsatz 4 verbunden werden. Vorliegend umfasst die Membran 7 hierzu aufsatzseitig Schnappkupplungen 11, welche in entsprechende Ausnehmungen am Aufsatz 4 einschnappen können. Aufsatz 4 ist in Fig. 13 und 14 im Detail dargestellt und beschrieben. Das Penetrationselement 6 umfasst eine Hohlnadel 9, welche sich vom Grundkörper 8 längs der Richtung H in Richtung zum Aufsatz 4 hin erstreckt. Ein innen liegender Fluidkanal 10 erstreckt sich längs der Hohlnadel 9 und mündet an einer mit Öffnungen versehenen Aufstechspitze 12 der Hohlnadel 9. Der Begriff Hohlnadel wird hier für jedes Element verwendet, das einen Ansaugkanal bildet und das in der Lage ist, das Verschlusselement zu penetrieren.

Wie insbesondere auch aus Fig. 3 und 4 ersichtlich ist, hat die Hohlnadel 9 eine relativ dicke Wandung, entlang welcher vorzugsweise parallele Belüftungskanäle 2 in der Form von Nuten angeordnet sind. Diese Nuten erstrecken sich vorzugsweise weitgehend über die gesamte Länge der Hohlnadel 9 von der Aufstechspitze 12 bis zum Grundkörper 8. Das gesamte Penetrationselement 6 ist vorzugsweise einstückig ausgebildet. Es kann in die Membran 7 eingeschweisst oder eingeschnappt sein oder es kann gar mit dieser ebenfalls einstückig ausgebildet sein. Die Belüftungskanäle 2 können im Querschnitt auch als Schwalbenschwanznut ausgebildet sein. Damit kann die Gefahr reduziert werden, dass das elastische Material des Verschlusselements die Belüftungskanäle 2 verschliesst.

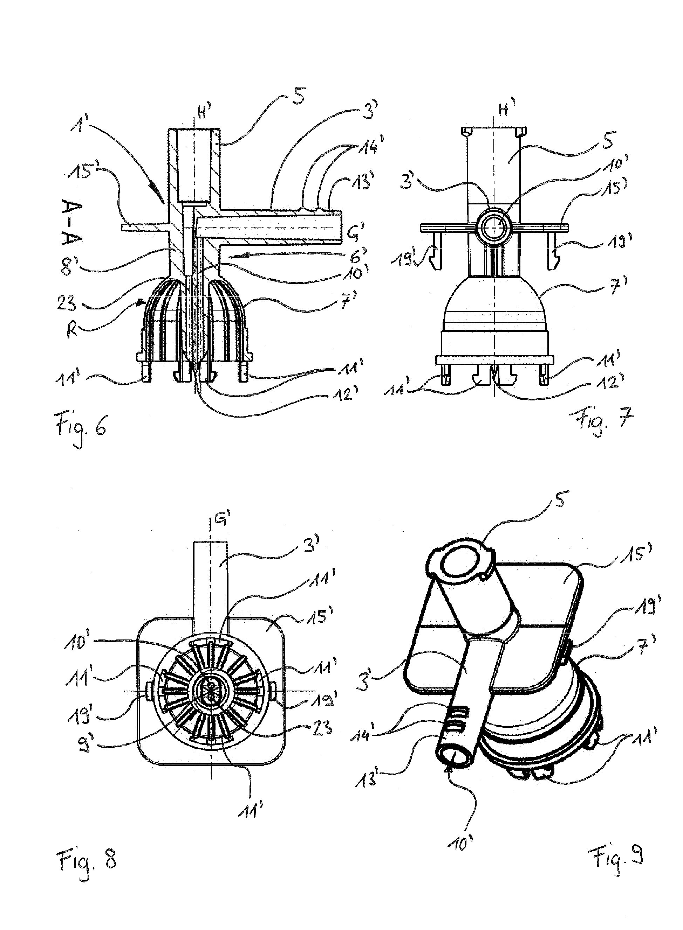

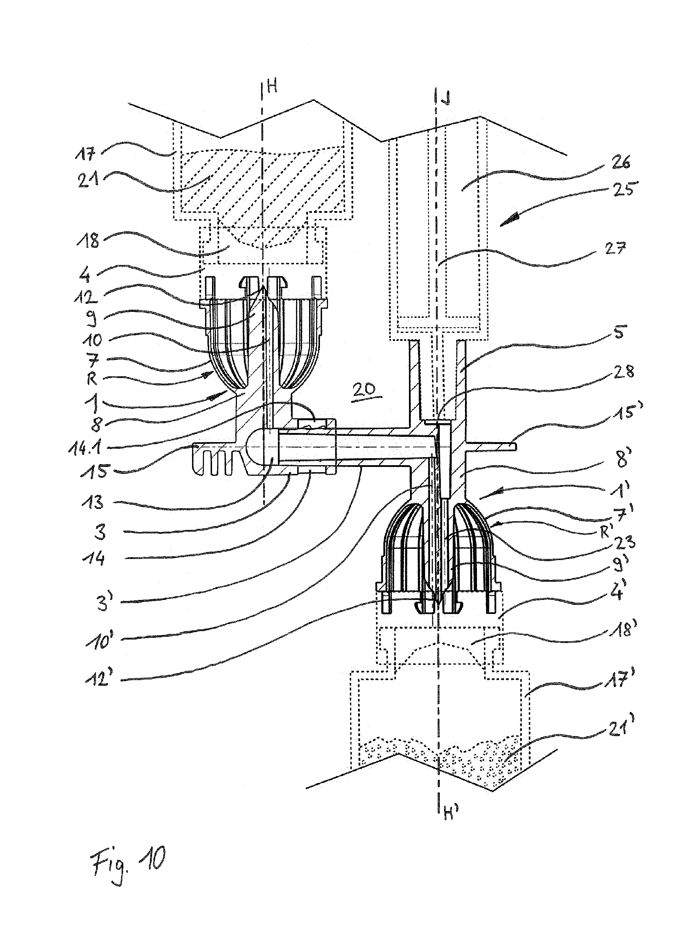

Weiter umfasst das Penetrationselement 6 den Anschlussstutzen 3, welcher sich in Richtung G senkrecht zu der Aufsatzrichtung H vom Grundkörper 8 weg erstreckt. Vorliegend ist der Anschlussstutzen 3 einstückig am Penetrationselement 6 angeformt. Der Anschlussstutzen 3 umfasst einen Aufnahmeraum 13 zur Ankopplung eines Anschlussstutzens 3' eines weiteren Adapters 1' (siehe Fig. 6 bis 9). Der Fluidkanal 10 der Hohlnadel 9 setzt sich bis zum Aufnahmeraum 13 fort und mündet in diesen. In einer Wandung des Anschlussstutzens 3 ist eine Rastausnehmung 14 für ein Rastmittel 14' des Anschlussstutzens 3' ausgebildet. Eine entsprechende Rastausnehmung 14.1 kann bezüglich G gegenüberliegend ausgebildet sein, sodass der Anschlussstutzen 23 in einer zweiten, um 180 Grad gedrehten, Ausrichtung im Aufnahmeraum 13 des Anschlussstutzens 3 verrasten kann (siehe Fig. 10).

Wie aus Fig. 2, 3 und 5 ersichtlich, umfasst das Penetrationselement 6 eine senkrecht zu H angeordnete Betätigungsplatte 15, in welcher der Anschlussstutzen 3 angeordnet ist. Die Betätigungsplatte 15 umfasst nach unten ragende Rastmittel 19, welche in einer Entnahmelage E des Penetrationselements 6 an entsprechenden Ausnehmungen des Aufsatzes 4 verrasten (siehe Fig. 3).

Die Membran 7 ist federelastisch ausgebildet, so dass sie in der dargestellten Ruhelage R das Penetrationselement 6 nach oben vorspannt. Innenseitig auf der Membran 7 sind Stabilisierungsleisten 16 ausgebildet, welche sich ausgehend vom Grundkörper 8 längs Meridianen auf der domartigen Membran 7 erstrecken und diese gezielt versteifen.

Nachstehend wird die Entnahme der Flüssigkeit aus einem an den Adapter 1 angeschlossenen Behälter 17 in der Entnahmelage E erläutert (nicht dargestellt). Zum Übergang in die Entnahmelage E wird eine Kraft in Richtung K auf das Penetrationselement 6 ausgeübt, z.B. über den Grundkörper 8 oder die Betätigungsplatte 15. Dabei wird das Penetrationselement 6 zum Aufsatz 4 hin bewegt, wobei sich die elastische Membran 7 deformiert. Gegebenenfalls platzt die Membran 7 dabei längs dafür vorgesehenen meridionaler Sollbruchlinien. Wird das Penetrationselement 6 vollständig zum Aufsatz 4 hin gedrückt, durchdringt die Hohlnadel 9 das Verschlusselement 18 des Behälters 17, so dass die Aufstechspitze 12 mit den dort ausgebildeten Öffnungen ins Innere des Behälters 17 hineinragt. Die Belüftungskanäle 2 schaffen dabei zusätzlich eine Verbindung zwischen dem Innenraum des Behälters 17 und einen von der Membran 7 begrenzten Innenraum.

In der Entnahmelage E wird die Membran 7 derart stark in Richtung Verschlusselement 18 gekrümmt, dass die Federvorspannung die Kraftrichtung ändert und das Penetrationselement 6 in der Entnahmelage E fixiert. Die Reibung zwischen der Hohlnadel 9 und dem Verschlusselement 18 trägt weiter dazu bei, dass das Penetrationselement 6 in dieser Position verbleibt. Zusätzlich verrasten die Rastmittel 19 am Aufsatz 4 und fixieren das Penetrationselement 6 in der Entnahmelage E.

Die eigentliche Entnahme der Flüssigkeit erfolgt nicht in der dargestellten Position, sondern kopfüber, so dass die Flüssigkeit im Behälter die Öffnung des Fluidkanals 10 an der Aufstechspitze 12 der Hohlnadel 9 flutet. Durch Erzeugung eines Unterdrucks im Aufnahmeraum 13 des Anschlussstutzens 3, z.B. durch Aufziehen einer angeschlossenen Spritze, kann somit Flüssigkeit aus dem Behälter 17 in den Aufnahmeraum 13 gesaugt werden. Der dabei im Behälter 17 entstehende Unterdruck über dem Flüssigkeitsspiegel wird ausgeglichen durch nachströmende Luft aus der Atmosphäre, welche über die Belüftungskanäle 2 in den Behälter 17 gelangt.

In den Fig. 6 bis 9 ist eine alternative Ausführungsform eines Adapters 1' dargestellt. Der besseren Übersichtlichkeit halber wurde in den Fig. 6 bis 9 auf eine Darstellung eines zugehörigen Aufsatzes 4' sowie eines angeschlossenen Behälters 17' verzichtet. Die Fig. 6 bis 9 sind im Folgenden gemeinsam beschrieben. Einander entsprechende Bauteile sind mit den gleichen Bezugszeichen mit Strich versehen.

An einem Grundkörper 8' eines Penetrationselements 6' ist der Anschlussstutzen 3' ausgebildet, welcher sich vom Anschlussstutzen 3' der Ausführungsform der Fig. 1 bis 6 unterscheidet. Der Anschlussstutzen 3' ist länger als der Anschlussstutzen 3 und weist keinen Aufnahmeraum auf. Vielmehr setzt sich ein Fluidkanal 10' einer Hohlnadel 9' längs des Anschlussstutzens 3' in dessen Längsrichtung, d.h. in Richtung G', fort, wobei ein Querschnitt des Fluidkanals 10' im Anschlussstutzen 3' aufgeweitet ist. Die Aussenmasse des Anschlussstutzens 3' in einem Endbereich 13' entsprechen weitgehend den Innenmassen des Aufnahmeraums 13 des Anschlussstutzens 3, sodass der Anschlussstutzen 3' in den Aufnahmeraum 13 nach aussen fluiddicht eingebracht werden kann. Der Anschlussstutzen 3' weist zudem Rastnasen 14' auf, welche zum Verrasten in die Aussparung 14 eingreifen können (siehe hierzu auch Fig. 10). Der Aufnahmeraum 13 des Anschlussstutzens 3 sowie der Endbereich 13' des Anschlussstutzens 3' bilden somit zueinander komplementäre Anschlusskupplungen der beiden Adapter 1 und 1'.

Am Grundkörper 8' ist nach oben ragend, d.h. in einer der Hohlnadel 9' entgegen gesetzten Richtung vom Grundkörper 8' weggerichtet, ein Innenkonus eines Luer-Locks 5 angeordnet. Es versteht sich, dass auch beliebig geartete Kopplungen für das Ankoppeln einer Abgabevorrichtung vorgesehen sein können. Der Innenkonus dient als Kopplungsmittel zum Ankoppeln einer Abgabevorrichtung wie beispielsweise einer Injektionsspritze (siehe z.B. Fig. 10). Das Kopplungsmittel gibt eine Kopplungsrichtung H', in welcher eine Abgabevorrichtung angekoppelt werden kann. Die Kopplungsrichtung H' fällt vorliegend mit einer Aufsatzrichtung eines Behälters 17' zusammen (siehe Fig. 10), ist allgemein bevorzugt parallel zu dieser angeordnet. Die Aufsatzrichtung entspricht in der Regel auch einer Längsrichtung sowohl des Behälters 17' als auch des Adapters 1'.

Im Innenkonus 5 mündet ein Fluidkanal 23, welcher sich bis an Öffnungen an der Aufstechspitze 12' der Hohlnadel 9' erstreckt. Die Hohlnadel 9' weist somit im Unterschied zu der Darstellung der Fig. 1 bis 5 keine Belüftungskanäle auf, jedoch zwei innen liegende Fluidkanäle 10' und 23.

Der Fluidkanal 23 dient hierbei als Entnahmekanal zur Entnahme einer Flüssigkeit aus einem an den Adapter 1' angeschlossenen Behälter 17' (siehe Fig. 10). Die eigentliche Entnahme der Flüssigkeit erfolgt hierbei ebenfalls nicht in der dargestellten Position, sondern kopfüber, so dass die Flüssigkeit im Behälter die Öffnung des Fluidkanals 23 an der Aufstechspitze 12' der Hohlnadel 9' flutet.

Figur 10 zeigt eine Transfervorrichtung 20, welche aus einem Adapter 1 und einem Adapter 1' zusammengesetzt ist. An den Adapter 1 ist am Aufsatz 4 ein Behälter 17 angeschlossen, welcher eine flüssige medizinische Substanz 21 enthält. An den Adapter 1' ist an einem Aufsatz 4' ein weiterer Behälter 17' angeschlossen, welcher ein feste medizinische Substanz 21' enthält.

Die beiden Adapter 1 und 1' sind über ihre Anschlussstutzen 3 bzw. 3' aneinander angeschlossen. Die Adapter 1 und 1' sind dabei mit den zugeordneten Aufsatzrichtungen H und H' antiparallel zueinander ausgerichtet, d.h. je nach Anordnung weist einer der angeschlossenen Behälter nach oben (Behälter 17) während der andere in die entgegen gesetzte Richtung nach unten weist (Behälter 17'). Hierzu sind die Adapter 1 und 1' derart aneinander angeschlossen, dass die Rastnasen 14' in der Rastausnehmung 14.1 verrastet sind.

An der Luer-Kupplung 5 ist eine Abgabevorrichtung 25 angekoppelt. Die Kopplungsrichtung H', in welcher die Abgabevorrichtung 25 in die Luer-Kupplung 5 eingebracht wird, entspricht dabei einer Längsrichtung J der Abgabevorrichtung 25. Ein in einem Innenraum 26 der Abgabevorrichtung 25 angeordneter und in Längsrichtung J fluiddicht verschiebbarer Stössel 27 ist ganz nach vorne zu einer Abgabeöffnung 28 der Abgabevorrichtung 25 hin verschoben, welche bei einer Mündung des Entnahmekanals 23 im Konus der Luer-Kupplung 5 angeordnet ist.

Die Behälter 17 und 17' sind versiegelt und die Penetrationselemente 6 und 6' der beiden Adapter 1 und 1' sind in der Ruhelage R bzw. R'. Die Transfervorrichtung 20 befindet sich somit in einem gebrauchsfertigen und lagerfähigen Zustand.

Zur Benutzung der Anordnung werden die Penetrationselemente 6 und 6' beider Adapter 1 und 1' in die Entnahmelage E bzw. E' (nicht dargestellt) gebracht, in welchen die Penetrationselemente 6 und 6' die jeweiligen Verschlusselemente 18 und 18' durchstossen. Wird sodann der Stössel 27 im Innenraum von der Abgabeöffnung 28 weg verschoben, wird über den Entnahmekanal 23 im Behälter 17' ein Unterdruck erzeugt. Aufgrund des Unterdrucks wird das flüssige Medikament 21 im Behälter 17, welches in der gezeigten Anordnung die Mündung des Fluidkanals 10 flutet, über den Fluidkanal 10 sowie den daran angeschlossenen Fluidkanal 10' in den Behälter 17' gesogen. Über den Belüftungskanal 2 kann dabei Luft aus der Atmosphäre in den Behälter 17 nachströmen.

Im Behälter 17' kann sich somit der Feststoff 21' in der Flüssigkeit 21 lösen. Ist der Auflösungsprozess abgeschlossen, kann die gesamte Vorrichtung kopfüber gestellt werden, sodass das Gemisch im Behälter 17' die Mündung des Entnahmekanals 23 an der Aufstechspitze 12' flutet. Durch weiteres Herausziehen des Stössels 27 kann das Gemisch in den Innenraum der Abgabevorrichtung 25 transferiert werden. Zur Benutzung kann die Ankopplung der Abgabevorrichtung 25 am Luer-Lock 5 gelöst werden und beispielsweise eine Injektionsnadel (nicht dargestellt) aufgesetzt werden.

Aus dem Obigen ist ersichtlich, dass im gesamten Transfer- und Mischprozess keine hygienisch heiklen Fluidverbindungen geschaffen werden müssen. Die spezifische Ausbildung der Adapter 1 und 1' mit den zwischen einer Ruhelage R zw. R' und einer Entnahmelage E bzw. E' verschiebbaren Penetrationselementen 6 und 6' erlaubt eine einfache und sichere Betätigung bzw. Aktivierung der gebrauchsfertigen Transfervorrichtung 20. Wie sich ebenso aus Fig. 10 ergibt, weist die Transfervorrichtung 20 mit daran angeschlossenen Behältern 17 und 17' aufgrund der quer ausgerichteten Anschlussstutzen 3 und 3' eine kompakte Baugrösse auf, insbesondere ist der Behälter 17 parallel zur Abgabevorrichtung angeordnet, welche beispielsweise zur Stabilisierung beim Transport an einer Schnappmanschette (nicht dargestellt) am Behälter 17 zusätzlich fixiert sein kann.

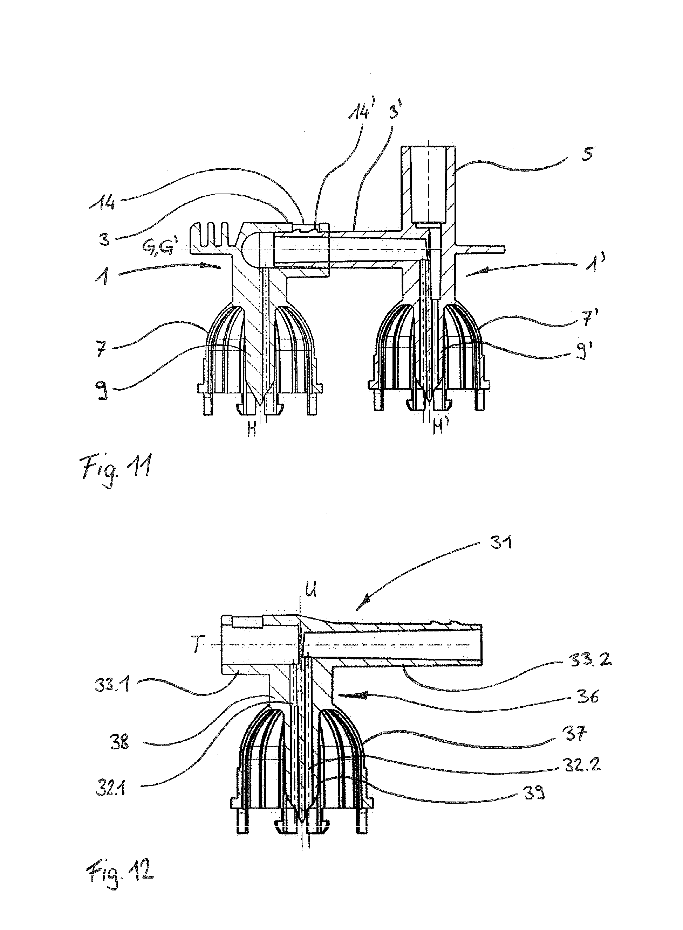

Figur 11 zeigt die Adapter 1 und 1' in einer alternativen Konfiguration, bei welcher die Aufsatzrichtungen H und H' parallel ausgerichtet sind (ohne Behälter, Aufsätze und Abgabevorrichtung). In dieser Konfiguration ist die Rastnase 14' in der Ausnehmung 14 angeordnet, womit sich eine bezüglich der Achse G/G' um 180 Grad gedrehte Anordnung der beiden Adapter 1 und 1' zueinander ergibt.

Diese Anordnung ist vorteilhaft für zwei Flüssigkeiten, welche direkt vor der Anwendung zu mischen sind. Zur Benutzung dieser Anordnung werden die Penetrationselemente 6 und 6' der parallel angeordneten Adapter 1 und 1' in die Entnahmelage E bzw. E' (nicht dargestellt) gebracht. Die eigentliche Entnahme der Flüssigkeiten erfolgt kopfüber, so dass die Flüssigkeiten in den Behältern 17 und 17' die Öffnungen des jeweiligen Fluidkanals 10 und 10' an den Aufstechspitzen 12 und 12' fluten. Wird sodann der Stössel 27 im Innenraum von der Abgabeöffnung 28 weg verschoben, wird über den Entnahmekanal 23 im Behälter 17' ein Unterdruck erzeugt. Die Flüssigkeit, welche aus dem Behälter 17' in die Abgabevorrichtung 25 transferiert wird, wird dabei mit Flüssigkeit aus dem Behälter 17 kompensiert, da nur beim Behälter 17 über den Belüftungskanal 2 Luft aus der Atmosphäre in den Behälter 17 nachströmen kann. Bei diesem Vorgang ergibt sich eine vorteilhafte Mischung der zwei Flüssigkeiten während des Transfers in die Abgabevorrichtung 25. Erst wenn Behälter 17 leer ist, beginnt sich Behälter 17' zu leeren, da nun Luft aus der Atmosphäre über den Behälter 17 in den Behälter 17' nachströmen kann. Nach dem vollständigen Transfer der nun gemischten Flüssigkeiten in den Innenraum der Abgabevorrichtung kann zur Benutzung die Ankopplung der Abgabevorrichtung 25 am Luer-Lock 5 gelöst werden und beispielsweise eine Injektionsnadel (nicht dargestellt) aufgesetzt werden.

Figur 12 zeigt eine mögliche weitere Ausführungsform eines erfindungsgemässen Adapters 31. Dieser weist zwei in einer Hohlnadel 39 eines Penetrationselements 36 ausgebildete innen liegende Fluidkanäle 32.1 und 32.2 auf. Das Penetrationselement 36 weist dabei einen Grundkörper 38 auf, an welchem ein erster Anschlussstutzen 33.1 und ein zweiter Anschlussstutzen 33.2 ausgebildet sind. Die Längsachsen der beiden Anschlussstutzen 33.1 und 33.2 liegen auf einer gemeinsamen Achse T, welche senkrecht zu einer Aufsatzrichtung U des Adapters 31 angeordnet sind. Es versteht sich, dass die Anschlussstutzen 33.1 und 33.2 nicht auf einer gemeinsamen Achse angeordnet sein müssen, sondern bezüglich U auch unter einem beliebigen Winkel wie z.B. 60 oder 90 Grad angeordnet sein können. Die Ausbildung einer elastischen Membran 37 als Führungsmittel des Penetrationselements 36 entspricht dabei z.B. der Membran 7 des Adapters 1.

Der Anschlussstutzen 33.1 ist analog dem Anschlussstutzen 3 des Adapters 1 ausgebildet, wobei ein Aufnahmeraum mit dem Fluidkanal 32.1 kommuniziert. Der Anschlussstutzen 33.2 ist hingegen entsprechend dem Anschlussstutzen 3' des Adapters 1' ausgestaltet, wobei sich der Fluidkanal 32.2 im Anschlussstutzen 33.2 fortsetzt. Adapter 33.1 bildet somit einen Zwischenadapter, welcher z.B. zwischen die Adapter 1 und 1' geschaltet werden kann, sofern ein weiteres Medikament benötigt wird.

Figuren 13 und 14 zeigen einen Längsquerschnitt durch einen Aufsatz 4 respektive eine Draufsicht auf eine Kopplungsseite zum Ankoppeln eines Behälters eines Aufsatzes 4.

Der Aufsatz 4 ist bevorzugt aus einem Kunststoffmaterial gefertigt, insbesondere einstückig als Formteil z.B. gespritzt, und weist einen rohrförmigen Grundkörper 70 auf. Der Aufsatz 4 weist einen Kopplungsbereich 71 zum Ankoppeln eines Behälters enthaltend eine flüssige Substanz auf, z.B. für den Behälter 17. Der Aufsatz 4 kann dabei mit dem Kopplungsbereich 71 nach Art einer Hülse auf eine entsprechend ausgebildete Behältermündung aufgesetzt werden. Im Kopplungsbereich 71 sind innenwandig am Grundkörper 70 Rastvorsprünge 72 ausgebildet, mit welchen der Aufsatz 4 nach Art eines Schnappkragens auf die Behältermündung aufgeschnappt werden kann. An der (normierten) Behältermündung ist ein nach aussen auskragender, umlaufender Randwulst 17.1 vorhanden, welchen die Rastvorsprünge 72 in aufgeschnapptem Zustand übergreifen und so den Aufsatz 4 am Behälter verrasten. Die Rastvorsprünge 72 sind derart dimensioniert und an die (normierte) Behältermündung angepasst, dass der Aufsatz 4 fest und ohne Spiel am Behälter gehalten ist und gegebenenfalls eine dichtende Presskraft auf ein Verschlusselement des Behälters ausüben kann.

Im rohrförmigen Grundkörper 70 ist ein in einer Ebene quer zur Längsachse S des Aufsatzes 4 angeordneter Zentrierring 73 mit einer zentralen Öffnung 74 ausgebildet. Der Zentrierring 73 grenzt im Innenraum des Grundkörper 70 dabei den zur Ankopplung eines Behälters vorgesehenen Kopplungsbereich 71 gegen einen Aufnahmebereich 76 für ein Penetrationselement mit Führungsmitteln wie beispielsweise das Penetrationselement 6 mit Membran 7 des Adapters 1 ab. In aufgeschnapptem Zustand des Aufsatzes 4 auf einem Behälter liegt der Zentrierring 73 kopplungsbereichsseitig dichtend an einem Verschlusselement des Behälters und/oder an der Behältermündung an, wobei das Verschlusselement beispielsweise zwischen einem Rand der Behältermündung und dem Zentrierring 73 eingeklemmt sein kann.

Der Zentrierring 73 weist Rastausnehmungen 77 auf, in welchen Schnappkupplungen der Führungsmittel eines Penetrationselements wie beispielsweise die Schnappkupplungen 11 eingeschnappt werden können, um so das Penetrationselement, z.B. das Penetrationselement 6, am Aufsatz zu befestigen. Die Führungsmittel, d.h. z.B. die Membran 7, weisen bevorzugt einen Auflagerand 7.1 auf, welcher in eingeschnapptem Zustand auf einem entsprechenden umlaufenden Vorsprung 78 am Zentrierring 73 aufliegt.

Die zentrale Öffnung 74 dient dabei zur Führung einer Hohlnadel z.B. der Hohlnadel 9, eines Penetrationselements, z.B. des Penetrationselements 6, wenn dieses von der Ruhelage R in die Entnahmelage E gebracht wird bzw. zur mechanischen Stabilisierung, wenn das Penetrationselement in der Entnahmelage E ist.

Kopplungsbereichseitig weist der Zentrierring 73 einen ringförmig um die zentrale Öffnung 73 ausgebildeten Aufnahmeraum 75 auf, in welchen in betriebsbereitem Zustand ein Filter (nicht dargestellt) eingesetzt ist. Der Aufnahmeraum 75 kommuniziert über Ventilationsöffnung 79 mit dem Anschlussbereich 76, sodass dem Aufnahmebereich 75 durch die Ventilationsöffnungen 79 Luft aus der Atmosphäre zuführbar ist. In aufgeschnapptem Zustand des Aufsatzes 4 auf einem Behälter liegt der Zentrierring 73 derart dichtend am Verschlusselement und/oder an der Behältermündung an, dass der Aufnahmeraum 75, abgesehen von den Ventilationsöffnungen 79 und der zentralen Öffnung 74, nach aussen weitgehend luftdicht abgeschlossen ist.

Befindet sich das Penetrationselement 6 in der Entnahmelage E, schliesst der aussenseitig als Nut an der Hohlnadel 9 ausgebildete Belüftungskanal 2 an den Aufnahmeraum 75 an, wobei die zentrale Öffnung 73 von der darin angeordneten Hohlnadel 9 und/oder dem Grundkörper 8 des Penetrationselement 6 weitgehend luftdicht abgedichtet ist. Dem Aufnahmeraum 75 und dem Belüftungskanal 2 kann somit in der Entnahmelage E des Penetrationselements 6 nur über die Ventilationsöffnungen 79 und somit über das darin angeordnete Filter Luft aus der Atmosphäre zugeführt werden. Damit wird sichergestellt, dass Luft, welche über den Belüftungskanal 2 in den angeschlossenen Behälter gelangt, zuerst das Filter passieren muss und somit von Keimen und Mikroorganismen sowie Schmutz befreit wird.

Im Anschlussbereich 76 sind am Grundkörper 70 zudem Rastausnehmungen 80 ausgebildet, in welchen entsprechend ausgebildete Rastmittel des Penetrationselements, z.B. die Rastmittel 19 des Penetrationselements 6, verrasten können, wenn das Penetrationselement 6 in der Entnahmelage E ist.

Es versteht sich, dass bei entsprechenden Aufsätzen für Adapter ohne Belüftungskanal auf das Filter verzichtet werden kann. Insbesondere kann der Aufsatz 4' für einen Adapter 1' oder 31 weitgehend baugleich ausgebildet sein, ohne dass ein Filter eingesetzt ist.

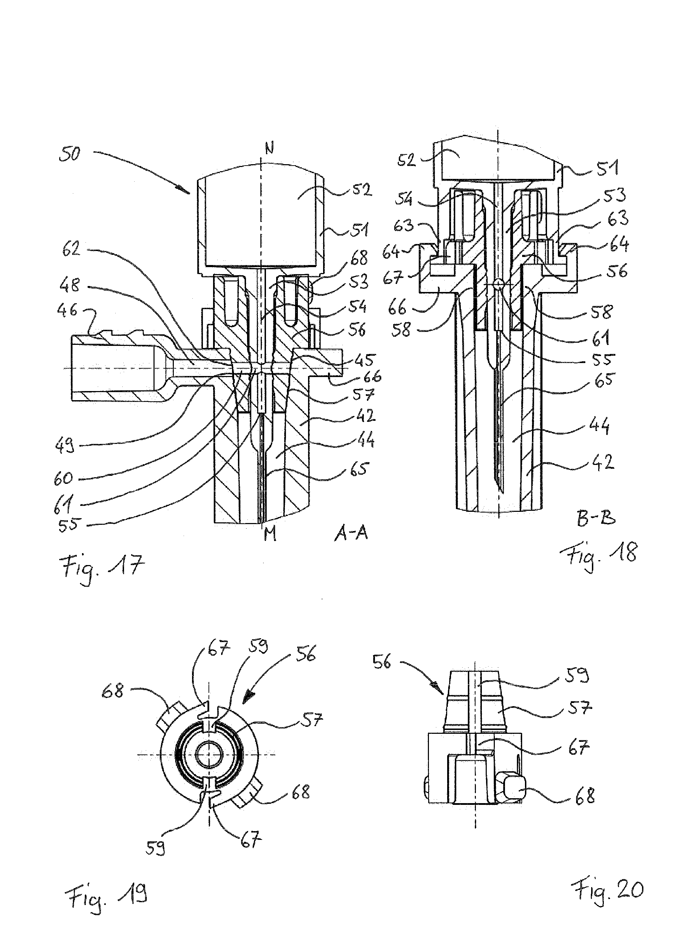

Figur 15 zeigt eine Schnittansicht eines nicht erfindungsgemäßen Adapters 41 in der Ebene A gemäss Fig. 16, welcher nur für die Zuführung eines Fluids zu einer ankoppelbaren Abgabevorrichtung (nicht dargestellt; siehe Fig. 17 und 18) vorgesehen ist. Figur 16 zeigt eine Draufsicht in Richtung der Längsachse M von einer Kopplungsseite her.

Der Adapter 41 umfasst ein längliches Gehäuse 42, welches einen in Längsrichtung M an einer Zugangsöffnung 43 offenen Aufnahmeraum 44 für eine Nadel oder Kanüle der Abgabevorrichtung aufweist. In einem Bereich an der Öffnung 43 ist ein als Kopplungsmittel ausgebildeter, weitgehend konischer Sitz 45 vorhanden. In einem Längsbereich bei der Öffnung 43 ist senkrecht zur Längsrichtung M ein Anschlussstutzen 46 ausgebildet, welcher einen analog dem Endbereich 13' des Adapters 1' als Anschlusskupplung ausgebildeten Endbereich 47 aufweist. Der Adapter 41 ist somit analog dem Adapter 1' zum Anschluss an den Anschlussstutzen 3 z.B. des Adapters 1 geeignet. Aussenseitig ist im Bereich des Anschlussstutzens 46 eine Halteplatte 66 ausgebildet, welche in einer Ebene senkrecht zu M flanschartig nach aussen auskragt und in deren Ebene der Anschlussstutzen 46 angeordnet ist.

Im Anschlussstutzen 46 ist ein am Anschlussstutzen 46 offener Fluidkanal 48 ausgebildet, welcher auf einer Mantelfläche des Sitzes 45 an einer Öffnung 49 mündet. Über den Fluidkanal 48 kann somit ein Fluid vom Anschlussstutzen 46 zum Sitz 45 transferiert werden.

Figur 17 zeigt einen Ausschnitt der Ansicht gemäss Fig. 15, wobei eine Abgabevorrichtung 50 im Adapter 41 angeordnet ist. Die Abgabevorrichtung 50 befindet sich dabei in einer Füllstellung, in welcher sie über den Adapter 41 mit einem Fluid befüllt werden kann. Figur 18 zeigt einen Längsschnitt des weitgehend selben Ausschnitts in einer zur Schnittebene der Ansichten gemäss Fig. 15 und 17 senkrechten Ebene (Ebene B gemäss Fig. 16).

Die Abgabevorrichtung 50 umfasst einen länglichen Grundkörper 51 (Längsachse N) mit einem Innenraum 52 zur Aufnahme eines Fluids. Stirnseitig ist ein Anschlusszapfen 53 am Grundkörper 51 ausgebildet, welcher sich in Längsrichtung N erstreckt und einen innen liegenden Fluidkanal 54 aufweist. Der Fluidkanal 54 verbindet den Innenraum 52 mit einer Abgabeöffnung 55 des Zapfens 53, an welcher eine Injektionsnadel 65 angebracht ist. Die Injektionsnadel 65 ist im Aufnahmeraum 44 angeordnet. Die Längsrichtung M des Adapters 41 und die Längsrichtung N sind in angekoppeltem Zustand im Wesentlichen koaxial angeordnet.

Auf dem Anschlusszapfen 53 ist eine ringförmige Manschette 56 bezüglich des Grundkörpers 51 um die Längsachse N rotierbar gelagert. Die Manschette 56 (in den Fig. 19 und 20 alleine dargestellt) weist einen weitgehend komplementär zum Sitz 45 ausgebildeten konischen Abschnitt 57 auf, mit welchem sie im Sitz 45 angeordnet ist. Im Sitz 45 sind in Längsrichtung N ausgerichtete Rippen 58 ausgebildet, welche in entsprechende Nuten 59 der Manschette 56 eingreifen und diese so gegen ein Verdrehen sichern.

Die Manschette 56 weist im Bereich der Öffnung 49 des Adapters 41 eine als Fluidkanal wirkende Querbohrung 60 auf, welche in der Füllstellung mit einer entsprechenden Querbohrung 61 im Anschlusszapfen 53 fluchtet und mantelseitig an der Manschette 56 an einer Füllöffnung 62 mündet. Die Füllöffnung 62 schliesst in der Füllstellung fluiddicht an den Fluidkanal 48 des Adapters 41 an. Die Querbohrung 61 kommuniziert mit dem Fluidkanal 54 und stellt somit in der Füllstellung einen durchgehenden Fluidkanal von der Füllöffnung 62 zum Innenraum 52 der Abgabevorrichtung her.

Vom Grundkörper 51 erstreckt sich, mantelseitig von der Manschette 56 beabstandet, ein Kopplungselement 63 in Längsrichtung N (siehe Fig. 18 und 21 bis 23). Am Adapter 41 sind hierzu komplementäre Kopplungselemente 64 ausgebildet, welche mit dem Kopplungselement 63 in der dargestellten Füllstellung bajonettartig im Eingriff sind.

Ist die Abgabevorrichtung 50 befüllt, wird der Grundkörper 51 in einer Entkopplungsrichtung bezüglich des Adapters 41 um die Längsachse N gedreht, um den Eingriff der Kopplungselemente 63 und 64 zu lösen. Dabei wird auch der Anschlusszapfen 53 mitgedreht, wobei die verdrehgesicherte Manschette 56 bezüglich des Adapters 41 in Ruhe bleibt. Die Querbohrungen 60 und 61 werden damit gegeneinander verdreht, womit ein Fluidschluss unterbrochen wird. Die Manschette 56 wirkt somit als Hahnsystem zum Verschliessen der Füllöffnung 62 gegenüber dem Innenraum 52. Bei ausreichender Drehung des Grundkörpers 51 wird auch der Eingriff der Kopplungselemente 63 und 64 gelöst, sodass die Abgabevorrichtung 50 in Längsrichtung N aus dem Adapter 41 abgezogen werden kann.

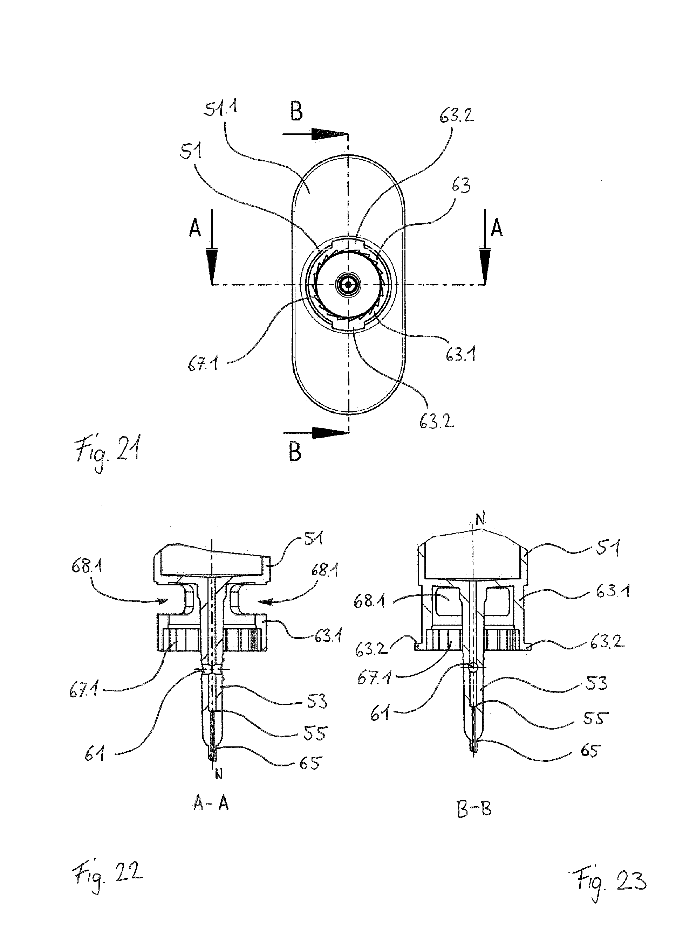

Um sicherzustellen, dass die Abgabevorrichtung 50 nur zum einmaligen Gebrauch geeignet ist und nicht erneut angekoppelt werden kann, weist die Manschette 56 Rastzungen 67 auf, welche mit Rastkerben 67.1 des Kopplungselements 63 derart zusammenwirken, dass nach Art einer Sperrklinke eine Drehung entgegen der Entkopplungsrichtung verhindert ist. Zudem sind umfangseitig an der Manschette 56 Anschläge 68 ausgebildet, welche in Aussparungen 68.1 der Kopplungsmittel 63 angeordnet sind und sicherstellen, dass der Grundkörper 51 nur in einem begrenzten Bereich bezüglich der Manschette 56 drehbar ist, insbesondere in Entkopplungsrichtung (siehe hierzu auch Fig. 21 bis 23). Der durch die Aussparungen 68.1 vorgegebene begrenzte Bereich ist dabei derart gewählt, dass die Abgabevorrichtung 50 nach einer den begrenzten Bereich ausschöpfenden relativen Drehung in Entkopplungsrichtung Manschette 56 und Grundkörper 51 in einer Stellung arretiert sind, welche eine erneute Ankopplung der Abgabevorrichtung 50 am Adapter 41 verhindert. Damit ist sichergestellt, dass die Abgabevorrichtung 50 nur einmal befüllt und benutzt werden kann.

Figur 21 zeigt eine Ansicht des Grundkörpers 51 der Abgabevorrichtung 50 in Richtung von N auf den Abgabebereich ohne Manschette 56. Figuren 22 und 23 zeigen Ausschnittsansichten von Längsschnitten im Bereich des Abgabebereichs der Abgabevorrichtung 50 in den Ebenen A bzw. B gemäss Fig. 21, ebenfalls ohne Manschette 56.

Das Kopplungselement 63 umfasst einen koaxial zur Längsrichtung N angeordneten, im Wesentlichen kreiszylindrischen Rohrabschnitt 63.1. Der Rohrabschnitt 63.1 weist im Wesentlichen dieselbe Aussendimension wie der Grundkörper 51 auf und erstreckt sich im Abgabebereich in Längsrichtung N vom Grundkörper 51 weg. In einem Innenraum des Rohrabschnitts 63.1 ist koaxial der Anschlusszapfen 53 angeordnet. Bei aufgesetzter Manschette 56 ist diese teilweise im Innenraum des Rohrabschnitts 63.1 angeordnet.

An einem dem Abgabebereich gegenüberliegenden Längsende des Grundkörpers 51 ist ein im Wesentlichen nach Art bekannter Spritzen ausgebildeter Fingerflansch 51.1 vorhanden.

In einem grundkörperfernen Endbereich weist der Rohrabschnitt 63.1 aussenseitig zwei flanschartig nach aussen auskragende, bezüglich der Längsrichtung N gegenüberliegend angeordnete Flügel 63.2 auf. Die Flügel 63.2 bilden dabei bajonettartige Eingriffsmittel, welche in der oben erwähnten Füllstellung mit den entsprechend ausgebildeten Kopplungselementen 64 am Adapter 41 im Eingriff sind.

Ebenfalls im grundkörperfernen Endbereich sind innenwandig am Rohrabschnitt 63.1 azimutal umlaufend die oben genannten Rastkerben 67.1 vorhanden. Die Rastkerben 67.1 sind dabei derart ausgebildet und angeordnet, dass bei eingesetzter Manschette 56 deren Rastzungen 67 mit den Rastkerben 67.1 nach Art einer Sperrklinke zusammenwirken, welche gegen eine relative Drehung von Grundkörper 51 und Manschette 56 entgegen der Entkopplungsrichtung sperrt.

Der Rohrabschnitt 63.1 weist in bezüglich N radialer Richtung zwei Durchbrüche auf. Die Durchbrüche bilden dabei die Aussparungen 68.1, in welchen die Anschläge 68 der Manschette 56 angeordnet sind, wenn diese auf den Grundkörper 51 aufgesetzt ist. Die Aussparungen 68.1 erstrecken sich dabei über eine vorgegebenen azimutalen Winkelbereich, welcher die relative Drehbarkeit zwischen Grundkörper 51 und Manschette 56 auf einen begrenzten Bereich einschränkt.

Es versteht sich, dass das Kopplungsmittel eines im Wesentlichen gemäss Adapter 1' ausgebildeten Adapters derart ausgebildet sein kann, dass eine Abgabevorrichtung gemäss der Abgabevorrichtung 50 angekoppelt und über den Entnahmekanal befüllt werden kann. Hierzu kann ein Gehäuse gemäss Gehäuse 42 beispielsweise seitlich versetzt an einem derartigen Adapter ausgebildet sein, wobei der Entnahmekanal an eine Öffnung auf der Mantelfläche des konischen Sitzes 45 geführt ist. Dem Fachmann erschliesst sich unmittelbar, wie eine derartige Ausführungsform umgesetzt werden kann.

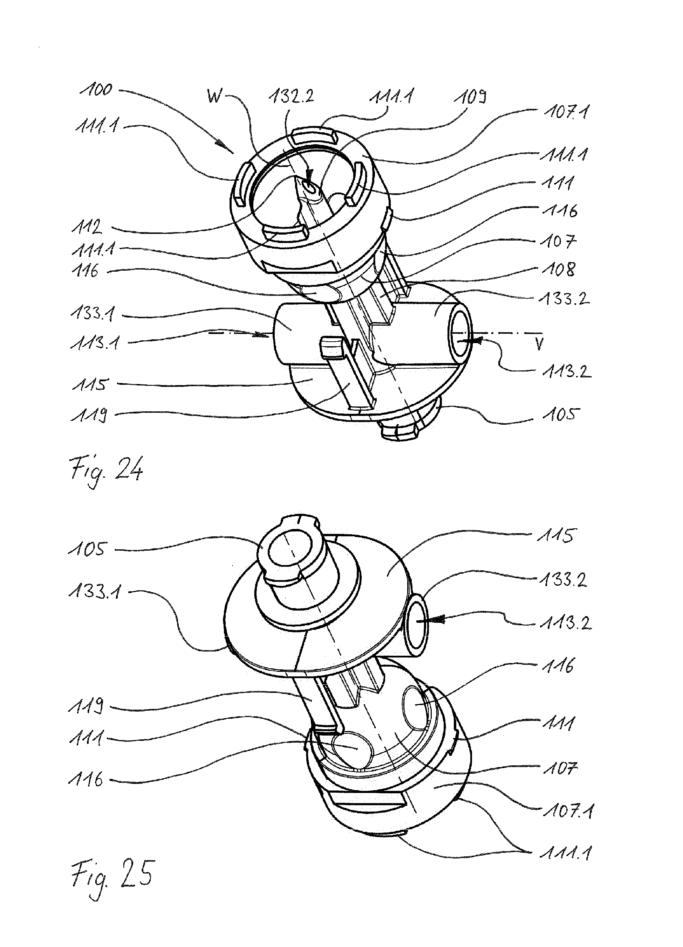

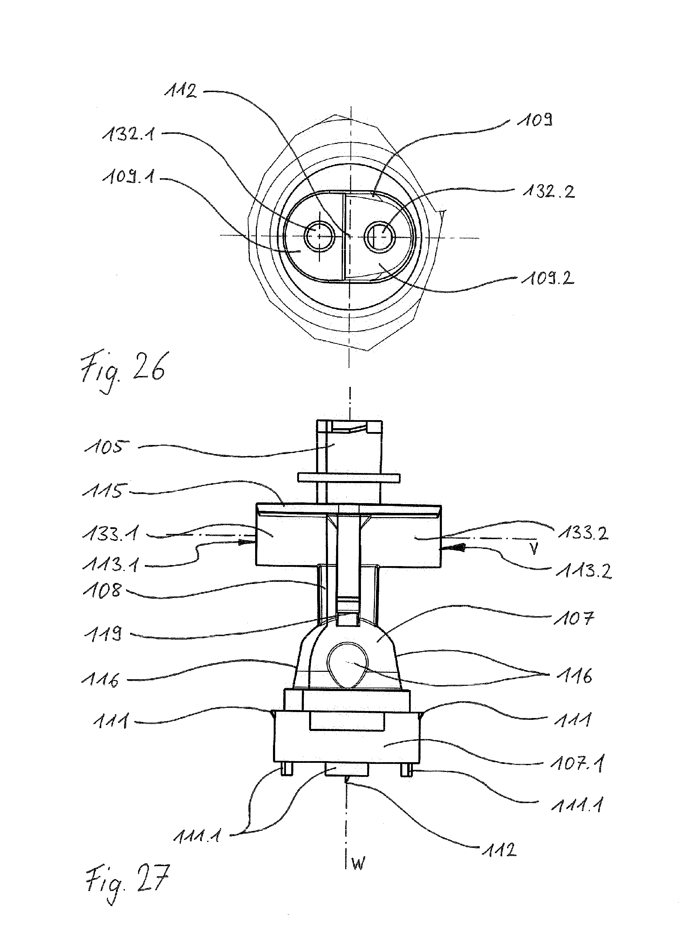

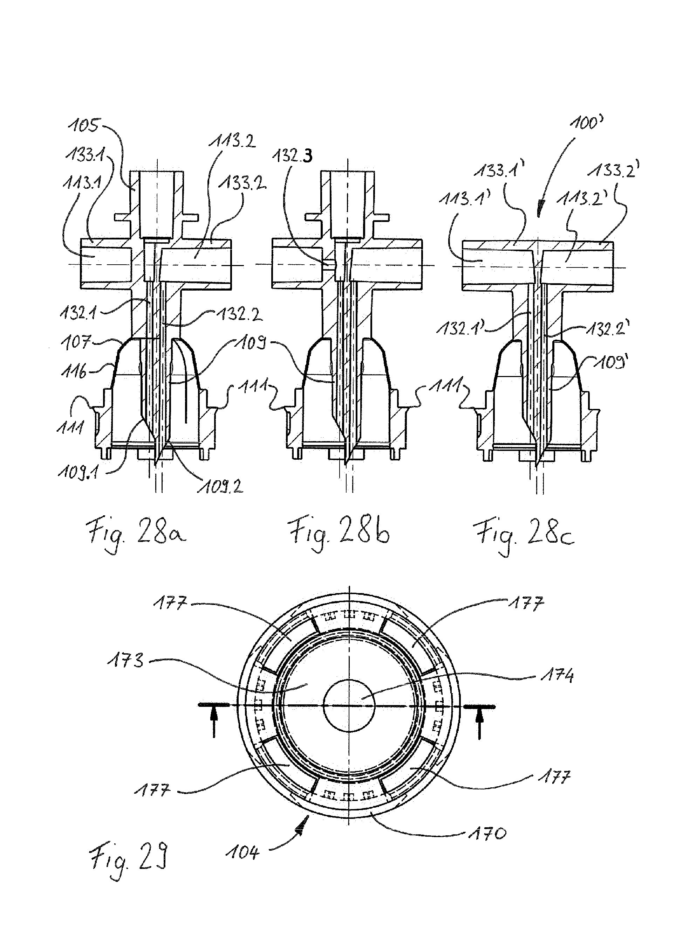

Figuren 24 bis 27 zeigen verschiedene Ansichten eines weiteren Ausführungsbeispiels eines Adapters 100 einer erfindungsgemässen Vorrichtung ohne Aufsatz 104 (siehe hierzu Fig. 29-31). Figuren 24 bis 27 sind im Folgenden gemeinsam beschrieben.

Der Adapter 100 weist eine Membran 107 auf (weitgehend analog der Membran 7, 7', 37), welche zur Verbindung des Penetrationselements 106 (weitgehend analog dem Penetrationselement 6, 6', 36) mit einem Aufsatz 104 (siehe Fig. 29-31) vorgesehen ist. Die Membran 107 ist kuppelartig ausgebildet und weist in einem zur Befestigung am Aufsatz 104 vorgesehenen Bereich einen Befestigungsring 107.1 auf. Der Befestigungsring 107.1 ist zur Befestigung der Membran 107 (und damit des Perforationselements 106) am Aufsatz 104 vorgesehen. Der Befestigungsring 107.1 ist derart ausgebildet, dass er in einem Anschlussbereich 176 des Aufsatzes 104 aufgenommen werden kann. Zur Befestigung am Aufsatz 104 weist der Befestigungsring 107.1 zwei bezüglich der Aufsatzrichtung W des Adapters 100 gegenüberliegend angeordnete, radial nach aussen auskragende Rastvorsprünge 111 auf. Diese können in entsprechende Rastausnehmungen 180 am Aufsatz 104 (siehe Fig. 29-31) verrastend eingreifen. Zudem sind stirnseitig am Befestigungsring 107.1 vier axiale Vorsprünge 111.1 angeordnet, welche zur Ausrichtung des Penetrationselements 106 axial in entsprechende Ausnehmungen 177 des Aufsatzes 104 (siehe Fig. 29-31) eingreifen.

Die Membran 107 weist vier im Wesentlichen kreisförmige Abflachungen 116 auf, welche z.B. von einer Sollbruchstelle begrenzt sein können. Gegebenenfalls platzt die Membran 107 beim Übergang in die Entnahmelage längs dieser Sollbruchlinien auf. Sofern ein Aufplatzen gewünscht ist, um z.B. erforderlichenfalls eine Luftzufuhr sicherzustellen, kann die Membran 107 innenwandig zwischen den Abflachungen 116 zusätzlich steife Perforationsstege aufweisen (nicht dargestellt).

An die Membran 107 schliesst ein im Wesentlichen zylindrischer Grundkörper 108 des Penetrationselements 106 an, welcher sich ausserhalb der Membran 107 in Längsrichtung fortsetzt. Der Grundkörper 108 kann z.B. einen kreisförmigen oder, wie in den Fig. 24 und 25 dargestellt, einen kreuzförmigen Querschnitt haben. Am Grundkörper 108 sind bezüglich der Aufsatzrichtung W ein erster Anschlussstutzen 133.1 und ein zweiter Anschlussstutzen 133.2 ausgebildet. Die Längsachsen der beiden Anschlussstutzen 133.1 und 133.2 liegen auf einer gemeinsamen Achse V, welche senkrecht zu der Aufsatzrichtung W des Adapters 100 angeordnet ist.

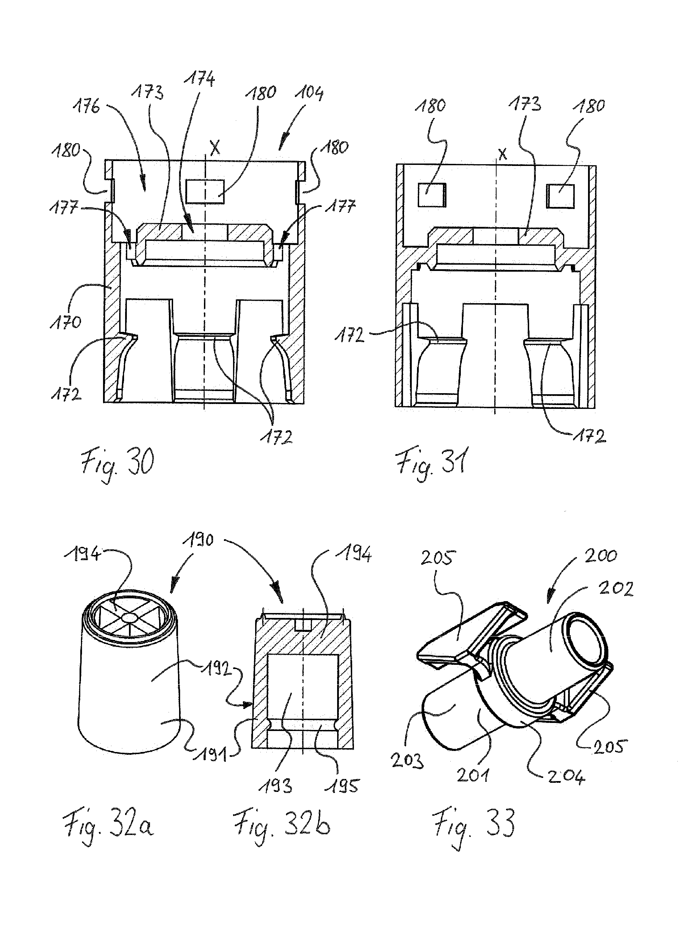

Jeder der Anschlussstutzen 133.1 und 133.2 weist einen Aufnahmeraum 113.1 bzw. 113.2 zur direkten oder indirekten Ankopplung eines weiteren erfindungsgemässen Adapters auf. Die Aufnahmeräume 113.1 und 113.2 weisen bevorzugt einen Innenkonus als Kopplungssitz auf. Auf diese Weise kann ein entsprechender Aussenkonus eines weiteren Adapters oder eines Kopplungsstücks 200 (siehe Fig. 33) auf einfache Weise fluiddicht an die Anschlussstutzen 133.1 und 133.2 angeschlossen werden. Ebenso kann z.B. ein Filtereinsatz 190 in die Aufnahmeräume 113.1 und 113.2 eingesetzt werden, sofern der entsprechende Anschlussstutzen als Belüftungsöffnung dienen soll.

Endseitig geht der Grundkörper 108 in eine Anschlusskupplung 105 eines Luer-Locks über. Zwischen Grundkörper 108 und Luer-Kupplung 105 kragt flanschartig eine Fingerplatte 115 quer zur Längsrichtung des Penetrationselements 106 aus. Eine Fläche der Fingerplatte 115 ist dabei grösser ausgebildet, als ein Grundriss der Membran 107, so dass in einer Draufsicht in Längsrichtung die gesamte Membran 107 mit Befestigungsring 107.1 von der Fingerplatte 115 überdeckt ist.

Die Luer-Kupplung 105 ist mit einem ersten Fluidkanal 132.1 verbunden. Der Fluidkanal 132.1 verläuft im Inneren des Grundkörpers 108 und einer Hohlnadel 109 des Penetrationselements 106 (siehe Fig. 28a- 28b). Die Hohlnadel 109 ist zentral innerhalb der kuppelförmigen Membran 107 angeordnet und erstreckt sich in Längsrichtung des Penetrationselements 106. Der erste Fluidkanal 132.1 mündet in einem Bereich an einer Aufstechspitze 112 der Hohlnadel 109 und ist bezüglich einer Längsachse der Hohlnadel 109 exzentrisch angeordnet. Auf diese Weise kann für eine gute Penetrationsfähigkeit die zentrale Aufstechspitze 112 massiv ausgebildet sein.

An der Fingerplatte 115 sind zwei bezüglich der Längsrichtung des Penetrationselements 106 gegenüberliegend angeordnete Rastzungen 119 ausgebildet, welche sich in Aufsatzrichtung W beidseitig der Membran 107 in Richtung zum Befestigungsring 107.1 erstrecken. Die Rastzungen 119 dienen dabei der Verrastung des Penetrationselements 106 am Aufsatz 104 in der Entnahmelage und greifen hierfür in der Entnahmelage in zwei weitere der Ausnehmungen 180 am Aufsatz 104 ein (siehe Fig. 29 bis 31).

Bevorzugt ist das Penetrationselement 106 zusammen mit der Membran 107 und den übrigen in den Fig. 24 bis 28c dargestellten Elementen als einstückiger Formteil aus Kunststoff hergestellt, insbesondere gespritzt. Selbstverständlich sind auch mehrteilige Ausführungen denkbar.

Figur 28a zeigt einen Längsquerschnitt durch den Adapter 100 ohne Aufsatz 104. In der Ausführungsform der Fig. 28a ist ein weiterer Fluidkanal 132.2 in der Hohlnadel 109 ausgebildet, welcher fluidmässig mit dem Aufnahmeraum 113.2 des Anschlussstutzens 133.2 kommuniziert. Der zweite Fluidkanal 132.2 erstreckt sich im Inneren der Hohlnadel 109 in Längsrichtung bis zu einer Aufstechspitze 112 der Hohlnadel 109. Der zweite Fluidkanal 132.2 ist ebenfalls exzentrisch in der Hohlnadel 109 angeordnet und mündet ebenfalls in einem Bereich an der Aufstechspitze 112. Die Mündungsöffnungen der beiden Fluidkanäle 132.1 und 132.2 sind dabei auf Schrägflächen 109.1 und 109.2 der Aufstechspitze 112 angeordnet, welche in Längsrichtung der Hohlnadel 109 versetzt angeordnet sind. Insbesondere ist die Fläche 109.1 mit der Öffnung des Kanals 132.1 näher zum Grundkörper 108 hin versetzt. Auf diese Weise sind in der Entnahmelage die Mündungsöffnungen unterschiedlich tief in einem angeschlossenen Behälter angeordnet. Mit anderen Worten münden die Fluidkanäle 132.1 und 132.2 der Hohlnadel 109 im Behälter in unterschiedlicher Entfernung vom Verschlusselement des Behälters.

An den Anschlussstutzen 133.2 kann, wie bereits zuvor in Zusammenhang mit anderen erfindungsgemässen Adaptern beschrieben, direkt oder indirekt ein weiterer erfindungsgemässer Adapter angeschlossen werden (z.B. weitgehend analog Fig. 10 oder über ein Kopplungsteil 200 gemäss Fig. 33). In der Ausführungsform der Fig. 28a ist der Anschlussstutzen 133.1 als Blindstutzen ausgebildet und weist keine Fluidverbindung mit einem der Fluidkanäle 132.1 oder 132.2 auf. Der Adapter 100 gemäss Fig. 28a entspricht hinsichtlich der Fluidführung somit weitgehend dem Adapter 1' gemäss Fig. 6.

Der Adapter 100 gemäss Fig. 28a kann in einer alternativen Anwendung auch alleine, ohne weiteren angeschlossenen Adapter, zur Entnahme einer Flüssigkeit aus einem angeschlossenen Behälter zum Einsatz kommen. Zur Entnahme wird beispielsweise eine Spritze an die Luer-Kupplung 105 angeschlossen. Durch Aufziehen der Spritze kann ein Unterdruck im Behälter erzeugt werden, mit welchem die Flüssigkeit durch den Fluidkanal 132.1 aufgezogen werden kann. Durch den Fluidkanal 132.2 kann Aussenluft in den Behälter nachströmen. Um sicherzustellen, dass die nachströmende Luft nicht verschmutzt und/oder keimfrei ist, kann der Filtereinsatz 190 gemäss Fig. 32a und 32b in den Aufnahmeraum 113.2 des Anschlussstutzens 133.2 eingesetzt werden. Der Fluidkanal 132.1 wirkt in diesem Fall als Entnahmekanal während der Fluidkanal 132.2 als Belüftungskanal wirkt.

Indem die Mündungsöffnung des Fluidkanals 132.2 tiefer in den Behälter hineinreicht, als die Mündungsöffnung des Fluidkanals 132.1, kann verhindert werden, dass sich im Bereich der Mündungsöffnungen eine Luftblase bildet. Derartige Luftblasen könnten aufgrund der Oberflächenspannung des Lösungsmittels bzw. der Lösung im Behälter einen unerwünschten direkten Luftschluss zwischen den beiden Mündungsöffnungen ergeben. Generell ist es daher vorteilhaft, wenn die Mündung eines Belüftungskanals in Längsrichtung auf einer anderen Höhe im Behälter angeordnet ist, als eine Mündung eines Entnahme- oder Transferkanals, wobei die Mündung des Belüftungskanals bevorzugt weiter vom Verschlusselement entfernt im Behälter angeordnet ist, als die Mündung des Entnahmekanals bzw. Transferkanals.

Figur 28b zeigt einen Längsquerschnitt durch den Adapter 100 ohne Aufsatz 104. In Fig. 28b weist der Aufnahmeraum 113.1 des Anschlussstutzens 133.1 eine Fluidverbindung 132.3 mit dem Fluidkanal 132.1 auf. Wie sich aus einem Vergleich der Fig. 28a und 28b ergibt, kann der im Wesentlichen selbe Adapter 100 genutzt werden, um durch eine geringfügige Modifikation zwei unterschiedliche Fluidführungen und somit unterschiedliche Anwendungsmöglichkeiten des Adapters zu schaffen.

Beispielsweise kann die Luer-Kupplung 105 des Adapters 100 gemäss Fig. 28b mit einer Kappe oder einem Pfropfen verschlossen werden. Auf dieses Weise hat der Adapter 100 eine weitgehend analoge Funktionalität wie der in Fig. 12 beschriebene Adapter 31. Hierzu kann sowohl an den Anschlussstutzen 133.1 wie auch an den Anschlussstutzen 133.2 ein weiterer Adapter angeschlossen werden. Alternativ kann auch nur ein weiterer Adapter z.B. an den Anschlussstutzen 133.1 angeschlossen sein. Der Anschlussstutzen 133.2 kann dabei als Belüftungsöffnung zum Nachströmen von Luft dienen, wobei in diesem Fall bevorzugt der Filtereinsatz 190 in den Aufnahmeraum 113.2 eingesetzt ist. Der Fluidkanal 132.2 wirkt in diesem Fall als Belüftungskanal. Über die Luer-Kupplung 105 kann z.B. eine Spritze zum Einbringen eines Lösungsmittels angeschlossenen werden. Nach dem Einbringen des Lösungsmittels kann die Lösung aus dem Behälter über den Anschlussstutzen 133.1 zu dem angeschlossenen Adapter transferiert werden. Dabei kann die leere Spritze die Luer-Kupplung 105 verschliessen.

Figur 28c zeigt eine weitere Ausführungsform eines Adapter 100', welcher eine weitgehend analoge Funktionalität wie der in Fig. 12 beschriebene Adapter 31 aufweist. Die Fluidkanäle 132.1' und 132.2' des Adapters 100' kommunizieren dabei fluidmässig direkt mit den Aufnahmeräumen 113.1' bzw. 113.2'. Der Adapter 100' gemäss Fig. 28c weist keine Luer-Kupplung auf.

Die Anschlussstutzen 133.1 und 133.2 sowie die Fluidkanäle 132.1 und 132.2 des Adapters 100 können auf unterschiedliche Weise verbunden sein und zur Anwendung kommen. Dies hat wesentliche fertigungstechnische Vorteile, da unterschiedliche Adapter mit nur geringfügigen Änderungen auf einfache Weise gemäss einer einzigen Grundausführungsform ausgebildet sein können.

Figuren 29 bis 31 zeigen einen für das Penetrationselement 106 der Figuren 24 - 28c vorgesehenen Aufsatz 104 des Adapters 100 bzw. 100' und sind im Folgenden gemeinsam beschrieben.

Der Aufsatz 104 hat einen rohrförmigen Grundkörper 170 mit einem Kopplungsbereich 171 zur Anordnung einer Mündung eines Behälters, z.B. des Behälters 17/17'. Im Kopplungsbereich 171 ist innenwandig ein Schnappkragen mit vier nach innen vorstehenden Rastvorsprüngen 172 ausgebildet. Im rohrförmigen Grundkörper 170 ist ein in einer Ebene quer zu einer Längsachse X des Aufsatzes 104 angeordneter Zentrierring 173 mit einer zentralen Öffnung 174 ausgebildet. Der Zentrierring 173 grenzt im Innenraum des Grundkörpers 170 den zur Ankopplung des Behälters vorgesehenen Kopplungsbereich 171 gegen den Aufnahmebereich 176 für das Penetrationselement 106 ab.

Der Zentrierring 173 weist Ausnehmungen 177 auf, in welche die Vorsprünge 111.1 des Befestigungsrings 107.1 in axialer Richtung eingreifen, um eine axiale Ausrichtung des Penetrationselements 106 vorzugeben. Im Aufnahmebereich 176 sind mantelseitig vier in radialer Richtung nach aussen offene Rastausnehmungen 180 ausgebildet. In jeweils zwei gegenüberliegenden Rastausnehmungen 180 können die Rastvorsprünge 111 des Befestigungsrings 107.1 verrasten, um das Penetrationselement 106 gegen ein Abziehen vom Aufsatz 104 zu sichern. Die weiteren beiden gegenüberliegenden Rastausnehmungen 180 dienen zum Eingriff der Rastzungen 119 des Penetrationselements 106, wenn sich dieses in der Entnahmelage befindet. Im Gegensatz zu den Aufsätzen 4/4' ist beim Aufsatz 104 kein Aufnahmeraum für einen Filtereinsatz vorgesehen. In diesem Fall kann ein Belüftungskanal erforderlichenfalls von einem der Fluidkanäle 132.1 und 132.2 der Hohlnadel 109 bereitgestellt sein, wobei eine Filterung der Luft vorzugsweise über den separaten Filtereinsatz 190 erreicht wird.

Ein wesentlicher Vorteil sämtlicher erfindungsgemässen Varianten von Aufsätzen wie z.B. 4, 4' oder 104 besteht darin, dass diese ohne weiteres für Behälter mit einem Bördelrand von 13 mm oder 20 mm Durchmesser bzw. für Behälter mit Bördelrändern beliebigen Durchmessers ausgebildet sein können.

Figur 32a zeigt eine Aussenansicht des Filtereinsatzes 190. Figur 32b zeigt einen Längsschnitt durch den Filtereinsatz 190. Im Folgenden sind beide Figuren gemeinsam beschrieben.

Der Filtereinsatz 190 weist einen Grundkörper 191 mit einer konischen Mantelfläche 192 auf. Der Grundkörper 191 weist einen kreisförmigen Querschnitt auf. Die konische Mantelfläche ist derart bemessen, dass der Filtereinsatz 190 reibschlüssig in den konischen Sitz der Aufnahmeräume 113.1 und 113.2 des Adapters 100 bzw. 100' eingesetzt werden kann.

Der Grundkörper 191 weist einen Innenraum 193 auf, welcher an beiden Längsenden offen ist, um einen Durchgang von Luft zu ermöglichen. In den Innenraum 193 kann beispielsweise ein Filterkörper (nicht dargestellt) wie z.B. ein Sinterfilter eingesetzt werden. Bevorzugt wird jedoch am verjüngten Längsende des Grundkörpers 191 von aussen her eine Filterfolie (nicht dargestellt) auf die Öffnung aufgesiegelt wie z.B. verklebt oder (laser-)verschweisst. Zur Halterung des Filters und/oder zum Schutz vor mechanischer Einwirkung weist der Filtereinsatz 190 am verjüngten Ende ein kreuzförmiges Gitter 194 in der dort angeordneten Öffnung auf. Im Bereich der gegenüberliegenden Öffnung ist im Innenraum 193 ein nach innen auskragender Rastwulst 195 ausgebildet, welcher gegebenenfalls einen Filterkörper im Innenraum 193 hält.

Für sämtliche erfindungsgemässen Ausführungen können als Filter neben den gängigen Mikrofiltermembranen oder Mikrofilterkörpern vorteilhaft insbesondere z.B. PET-Folien oder Polykarbonat-Folien verwendet werden, welche von feinsten Öffnungen durchsetzt sind. Anstelle von separat eingelegten oder angeklebten Filtern könnten diese auch direkt beim Spritzgiessen der jeweiligen Tragkonstruktionen bzw. des Filtereinsatzes integriert werden.

Figur 33 zeigt das Kopplungsteil 200 zum indirekten Anschluss zweier erfindungsgemässer Adapter. Das Kopplungsteil 200 weist einen endseitig offenen rohrförmigen Grundkörper 201 auf, welcher aussenseitig in beiden Endbereichen einen konischen Abschnitt 202 bzw. 203 aufweist. Die konischen Abschnitte 202 und 203 sind durch einen aussen umlaufenden, ringförmigen Abstandhalter 204 gegeneinander abgegrenzt. Die konischen Abschnitte 202 und 203 sind derart bemessen, dass das Kopplungsteil 200 reibschlüssig in den konischen Sitz der Aufnahmeräume 113.1 und 113.2 der Adapter 100 bzw. 100' eingesetzt werden kann. Am Abstandshalter 204 sind Halteflügel 205 angeordnet, welche zur Handhabung sowie für eine allfällige Verrastung des Kopplungsteils 200 an den Anschlussstutzen 133.1 bzw. 133.2 ausgebildet sein können. Die Halteflügel 205 können zudem derart ausgebildet sein, dass bei Zusammenwirken mit entsprechenden Mitteln an den Anschlussstutzen 133.1 und 133.2 eine relative Ausrichtung zweier aneinander angeschlossenen Adapter vorgegeben ist.

## Patentansprüche

1. Adapter (1) für eine Transfervorrichtung (20) für ein Fluid, zum Anschluss eines Behälters (17) enthaltend das Fluid, wobei der Behälter (17) mit einem penetrierbaren Verschlusselement (18) verschlossen ist und der Adapter (1) einen dichtend in einer Aufsatzrichtung auf eine mit dem Verschlusselement verschlossenen Behältermündung aufsetzbaren Aufsatz (4) aufweist, an welchem ein Penetrationselement (6) derart gelagert ist, dass das Penetrationselement (6) mit Hilfe von Führungsmitteln zwischen einer Ruhelage (R) und einer Entnahmelage (E) verschiebbar ist, wobei das Penetrationselement (6) eine Hohlnadel (9) aufweist, mit welcher in der Entnahmelage (E) das Verschlusselement (18) penetrierbar ist, wobei der Adapter (1) einen Anschlussstutzen (3) mit einer Anschlusskupplung zum fluiddichten Anschluss eines weiteren Adapters (1') aufweist und ein Transferkanal (10) von einer Mündung am Anschlussstutzen (3) bis zu einer Mündung an der Hohlnadel (9) verläuft, sodass in der Entnahmelage (E) des Penetrationselements (6) die Mündung des Transferkanals (10) am Anschlussstutzen (3) mit einem Innenraum des angeschlossenen Behälters (17) kommuniziert,
**dadurch gekennzeichnet, dass**
der Anschlussstutzen (3) quer, insbesondere weitgehend senkrecht, zur Aufsatzrichtung (H), vorzugsweise am Penetrationselement (6), angeordnet ist.

2. Adapter (1) nach Anspruch 1, **dadurch gekennzeichnet, dass** die Führungsmittel wenigstens einen Wandabschnitt aufweisen, der das Penetrationselement (6) mit dem Aufsatz (4) verbindet und dieses in der Ruhelage (R) hält, wobei der Wandabschnitt derart verformbar ist, dass er das Penetrationselement (6) bis zum Erreichen der Entnahmelage (E) führt.

3. Adapter (1) nach Anspruch 2, **dadurch gekennzeichnet, dass** der Wandabschnitt wenigstens eine das Penetrationselement (6) umgebende, federelastische Membran (7) umfasst.

4. Adapter (1) nach einem der Ansprüche 1 bis 3, **dadurch gekennzeichnet, dass** ein Belüftungskanal (2) zum Belüften des Behälters (17) bei einer Entnahme des Fluids vorhanden ist.

5. Adapter (1) nach Anspruch 4, **dadurch gekennzeichnet, dass** der Belüftungskanal (2) eine Nut auf der Aussenseite der Hohlnadel (9) umfasst.

6. Adapter (1) nach Anspruch 4 oder 5, **dadurch gekennzeichnet, dass** er wenigstens ein Filter aufweist, über das in der Entnahmelage (E) Luft aus der Atmosphäre über den Belüftungskanal (2) in den Behälter (17) zuführbar ist.

7. Adapter nach Anspruch 6, **dadurch gekennzeichnet, dass** das Filter am Aufsatz (4), vorzugsweise in einem der Behältermündung zugewandten Bereich angeordnet ist, insbesondere in einer Halterung (75) angeordnet ist, welche das Penetrationselement (6) kreisringförmig umgibt, wenn dieses in der Entnahmelage (E) ist.

8. Adapter (1') gemäss einem der Ansprüche 1 bis 3, **dadurch gekennzeichnet, dass** ein Kopplungsmittel (5) zum Ankoppeln einer Abgabevorrichtung in einer Kopplungsrichtung (H') vorhanden ist und dass ein als Entnahmekanal (23) ausgebildeter weiterer Fluidkanal von einer Mündung am Kopplungsmittel bis zu einer Mündung an der Hohlnadel (9') verläuft, sodass in der Entnahmelage (E) des Penetrationselements (6') die Mündung des Entnahmekanals (23) beim Kopplungsmittel mit dem Innenraum eines angeschlossenen Behälters (17') kommuniziert, wobei bevorzugt die Kopplungsrichtung (H') parallel zur Aufsatzrichtung ausgerichtet ist und insbesondere das Kopplungsmittel (5) als Luer-Innenkonus (5) ausgebildet ist.

9. Adapter (31) gemäss einem der Ansprüche 1 bis 3, **dadurch gekennzeichnet, dass** ein zweiter Anschlussstutzen (33.1) mit einer Anschlusskupplung zum fluiddichten Anschluss eines weiteren Adapters (1, 1') vorhanden ist, und dass ein als zweiter Transferkanal (32.1) ausgebildeter weiterer Fluidkanal von einer Mündung am zweiten Anschlussstutzen (33.1) bis zu einer Mündung an der Hohlnadel (39) verläuft, sodass in der Entnahmelage des Penetrationselements (36) die Mündung des zweiten Transferkanals (32.1) am zweiten Anschlussstutzen (33.1) mit dem Innenraum eines angeschlossenen Behälters kommuniziert, wobei der zweite Anschlussstutzen (33.1) quer, insbesondere weitgehend senkrecht, zur Aufsatzrichtung (H) angeordnet ist.

10. Transfervorrichtung (20) für den Transfer eines Fluids aus einem Behälter (17) in eine Abgabevorrichtung, umfassend einen ersten Adapter (1) gemäss einem der Ansprüche 4 bis 8 und einen zweiten Adapter (1') gemäss Anspruch 8.

11. Transfervorrichtung (20) gemäss Anspruch 10, **dadurch gekennzeichnet, dass** die Anschlusskupplungen der beiden Adapter (1, 1') derart komplementär zueinander ausgebildet sind, dass der erste Adapter (1) an den zweiten Adapter (1') fluiddicht anschliessbar ist.

12. Transfervorrichtung (20) gemäss einem der Ansprüche 10 bis 11, **dadurch gekennzeichnet, dass** die Anschlusskupplungen der beiden Adapter (1, 1') derart ausgebildet sind, dass die Adapter (1, 1') in genau zwei Ausrichtungen aneinander koppelbar sind, wobei bevorzugt die Anschlussrichtung (H) des ersten Adapters (1) und die Kopplungsrichtung (H',M) des zweiten Adapters (1') in einer der beiden Ausrichtungen entgegengesetzt und in der anderen Ausrichtung gleichgerichtet sind, wobei vorzugsweise die Anschlusskopplungen zueinander komplementäre Rastmittel (14, 14') umfassen, welche insbesondere zum einen die Ausrichtungen der beiden Adapter (1, 1') vorgeben und vorzugsweise zum anderen die von den beiden Anschlusskopplungen hergestellte Verbindung der beiden Adapter (1, 1') sichern.

13. Transfervorrichtung (20) gemäss einem der Ansprüche 10 bis 12, **dadurch gekennzeichnet, dass** eine der Anschlusskupplungen der beiden Adapter (1, 1') einen Aussenkonus und die andere der beiden Anschlusskopplungen einen Innenkonus umfasst, wobei insbesondere der Innenkonus an der Anschlusskopplung des ersten Adapters (1) ausgebildet ist.

14. Transfervorrichtung gemäss einem der Ansprüche 10 bis 13, **dadurch gekennzeichnet, dass** wenigstens ein weiterer Adapter (31) vorhanden ist, welcher gemäss Anspruch 9 ausgebildet ist, wobei insbesondere die Anschlusskupplung des ersten Anschlussstutzens (33.2) komplementär zur Anschlusskupplung des ersten Adapters (1) und vorzugsweise die Anschlusskupplung des zweiten Anschlussstutzens (33.1) komplementär zur Anschlusskupplung des zweiten Adapters (1') ausgebildet sind, sodass der weitere Adapter (31) gleichzeitig fluiddicht direkt an den ersten (1) und den zweiten Adapter (1') anschliessbar ist.

15. Transfervorrichtung gemäss einem der Ansprüche 10 bis 14, **dadurch gekennzeichnet, dass** ein gemeinsames Gehäuse vorhanden ist, in welchem die Adapter (1, 1') der Transfervorrichtung, und gegebenenfalls eine Abgabevorrichtung, in einer für die Benutzung vorgesehenen Konfiguration angeordnet sind.

## Claims

1. An adapter (1) for a transfer device (20) for a fluid for connecting a container (17) containing the fluid, wherein the container (17) is sealed with a penetrable sealing element (18) and the adapter (1) has a cap member (4) which can be positioned in a sealing manner in a positioning direction on a container opening which is sealed with the sealing element and on which a penetration element (6) is supported in such a manner that the penetration element (6) can be displaced by means of guide means between a rest position (R) and a removal position (E), wherein the penetration element (6) has a hollow needle (9), with which the sealing element (18) can be penetrated in the removal position (E), wherein the adapter (1) has a connection piece (3) having a connection coupling for the fluid-tight connection of another adapter (1') and a transfer channel (10) extends from an opening in the connection piece (3) as far as an opening in the hollow needle (9) such that the opening of the transfer channel (10) in the connection piece (3) communicates with an inner space of the connected container (17) in the removal position (E) of the penetration element (6),
**characterized in that**
the connection piece (3) is arranged transversely, in particular substantially perpendicularly, relative to the positioning direction (H), preferably on the penetration element (6).

2. Adapter (1) according to Claim 1, **characterized in that** the guide means have at least one wall portion which connects the penetration element (6) to the cap member (4) and holds it in the rest position (R), wherein the wall portion can be deformed in such a manner that it guides the penetration element (6) until the removal position (E) is reached.

3. Adapter (1) according to Claim 2, **characterized in that** the wall portion comprises at least one resilient membrane (7) which surrounds the penetration element (6).

4. Adapter (1) according to one of Claims 1 to 3, **characterized in that** a ventilation channel (2) is provided for ventilating the container (17) when the fluid is removed.

5. Adapter (1) according to Claim 4, **characterized in that** the ventilation channel (2) comprises a groove at the outer side of the hollow needle (9).

6. Adapter (1) according to Claim 4 or 5, **characterized in that** it has at least one filter via which air can be supplied in the removal position (E) from the atmosphere to the container (17) via the ventilation channel (2).

7. Adapter according to Claim 6, **characterized in that** the filter is arranged at the cap member (4), preferably in a region facing the container opening, in particular arranged in a retention member (75) which surrounds the penetration element (6) in the manner of a circular ring if the penetration element (6) is in the removal position (E).

8. Adapter (1') according to one of Claims 1 to 3, **characterized in that** a coupling means (5) for coupling a discharge device in a coupling direction (H') is provided and **in that** an additional fluid channel, which is in the form of a removal channel (23), extends from an opening in the coupling means as far as an opening in the hollow needle (9') so that, when the penetration element (6') is in the removal position (E), the opening of the removal channel (23) in the coupling means communicates with the inner space of a connected container (17'), wherein the coupling direction (H') is preferably orientated parallel with the positioning direction and in particular the coupling means (5) is in the form of an internal Luer cone (5).

9. Adapter (31) according to one of Claims 1 to 3, **characterized in that** a second connection piece (33.1) having a connection coupling for the fluid-tight connection of another adapter (1, 1') is provided, and **in that** an additional fluid channel which is in the form of a second transfer channel (32.1) extends from an opening in the second connection piece (33.1) as far as an opening in the hollow needle (39) so that, when the penetration element (36) is in the removal position, the opening of the second transfer channel (32.1) in the second connection piece (33.1) communicates with the inner space of a connected container, wherein the second connection piece (33.1) is arranged transversely, in particular substantially perpendicularly, relative to the positioning direction (H).

10. Transfer device (20) for transferring a fluid from a container (17) to a discharge device, comprising a first adapter (1) according to one of Claims 4 to 8 and a second adapter (1') according to Claim 8.

11. Transfer device (20) according to Claim 10, **characterized in that** the connection couplings of the two adapters (1, 1') are constructed so as to complement each other in such a manner that the first adapter (1) can be connected to the second adapter (1') in a fluid-tight manner.

12. Transfer device (20) according to either of Claims 10 and 11, **characterized in that** the connection couplings of the two adapters (1, 1') are constructed in such a manner that the adapters (1, 1') can be coupled to each other in precisely two orientations, wherein the connection direction (H) of the first adapter (1) and the coupling direction (H', M) of the second adapter (1') are preferably opposed in one of the two orientations and are preferably aligned in the other orientation, wherein the connection couplings preferably comprise mutually complementary engaging means (14, 14') which particularly, on the one hand, predetermine the orientations of the two adapters (1, 1') and preferably, on the other hand, secure the connection of the two adapters (1, 1') produced by the two connection couplings.

13. Transfer device (20) according to one of Claims 10 to 12, **characterized in that** one of the connection couplings of the two adapters (1, 1') comprises an outer cone and the other of the two connection couplings comprises an inner cone, wherein particularly the inner cone is formed at the connection coupling of the first adapter (1).

14. Transfer device according to one of Claims 10 to 13, **characterized in that** there is provided at least one additional adapter (31) which is constructed according to claim 9, wherein in particular the connection coupling of the first connection piece (33.2) is constructed so as to correspond to the connection coupling of the first adapter (1) and the connection coupling of the second connection piece (33.1) is preferably constructed so as to correspond to the connection coupling of the second adapter (1') so that the additional adapter (31) can be connected simultaneously to the first adapter (1) and second adapter (1') directly in a fluid-tight manner.

15. Transfer device according to one of Claims 10 to 14, **characterized in that** there is provided a common housing in which the adapters (1, 1') of the transfer device and optionally a discharge device are arranged in a configuration provided for use.

## Revendications

1. Adaptateur (1) pour un dispositif de transfert (20) pour un fluide, pour le raccordement d'un récipient (17) contenant le fluide, le récipient (17) étant fermé avec un élément de fermeture perçable (18) et l'adaptateur (1) présentant un couvercle (4) pouvant être posé de manière hermétique dans une direction de pose sur une ouverture de récipient fermée par l'élément de fermeture, sur lequel couvercle est supporté un élément de perçage (6) de telle sorte que l'élément de perçage (6) puisse être déplacé à l'aide de moyens de guidage entre une position de repos (R) et une position de prélèvement (E), l'élément de perçage (6) présentant une aiguille creuse (9), avec laquelle l'élément de fermeture (18) peut être percé dans la position de prélèvement (E), l'adaptateur (1) présentant une tubulure de raccordement (3) avec un accouplement de raccordement pour le raccordement étanche aux fluides d'un adaptateur supplémentaire (1') et un canal de transfert (10) s'étendant depuis une ouverture au niveau de la tubulure de raccordement (3) jusqu'à une ouverture au niveau de l'aiguille creuse (9), de telle sorte que dans la position de prélèvement (E) de l'élément de perçage (6), l'ouverture du canal de transfert (10) au niveau de la tubulure de raccordement (3) communique avec un espace interne du récipient raccordé (17), **caractérisé en ce que**
la tubulure de raccordement (3) est disposée transversalement, notamment essentiellement perpendiculairement, à la direction de pose (H), de préférence sur l'élément de perçage (6).

2. Adaptateur (1) selon la revendication 1, **caractérisé en ce que** les moyens de guidage présentent au moins une portion de paroi qui relie l'élément de perçage (6) au couvercle (4) et le retient dans la position de repos (R), la portion de paroi étant déformable de telle sorte qu'elle guide l'élément de perçage (6) jusqu'à ce qu'il atteigne la position de prélèvement (E).

3. Adaptateur (1) selon la revendication 2, **caractérisé en ce que** la portion de paroi comprend au moins une membrane (7) élastique à ressort entourant l'élément de perçage (6).

4. Adaptateur (1) selon l'une quelconque des revendications 1 à 3, **caractérisé en ce qu'**un canal de ventilation (2) est prévu pour ventiler le récipient (17) lors d'un prélèvement du fluide.

5. Adaptateur (1) selon la revendication 4, **caractérisé en ce que** le canal de ventilation (2) comprend une rainure sur le côté extérieur de l'aiguille creuse (9).

6. Adaptateur (1) selon la revendication 4 ou 5, **caractérisé en ce qu'**il présente au moins un filtre par le biais duquel de l'air peut être acheminé dans la position de prélèvement (E) depuis l'atmosphère par le biais du canal de ventilation (2) jusque dans le récipient (17).

7. Adaptateur selon la revendication 6, **caractérisé en ce que** le filtre est disposé sur le couvercle (4), de préférence dans une région tournée vers l'ouverture de récipient, notamment dans une fixation (75) qui entoure de manière annulaire circulaire l'élément de perçage (6), lorsque celui-ci est dans la position de prélèvement (E).

8. Adaptateur (1') selon l'une quelconque des revendications 1 à 3, **caractérisé en ce qu'**un moyen d'accouplement (5) est prévu pour l'accouplement d'un dispositif de distribution dans une direction d'accouplement (H')., et **en ce qu'**un canal de fluide supplémentaire réalisé sous forme de canal de prélèvement (23) s'étend depuis une ouverture au niveau du moyen d'accouplement jusqu'à une ouverture au niveau de l'aiguille creuse (9'), de telle sorte que dans la position de prélèvement (E) de l'élément de perçage (6'), l'ouverture du canal de prélèvement (23) communique au niveau du moyen d'accouplement avec l'espace interne d'un récipient raccordé (17'), la direction d'accouplement (H') étant de préférence orientée parallèlement à la direction de pose, et notamment le moyen d'accouplement (5) étant réalisé sous forme de cône interne de Luer (5).

9. Adaptateur (31) selon l'une quelconque des revendications 1 à 3, **caractérisé en ce qu'**une deuxième tubulure de raccordement (33.1) est prévue avec un accouplement de raccordement pour le raccordement étanche aux fluides d'un adaptateur supplémentaire (1, 1'), et **en ce qu'**un canal de fluide supplémentaire réalisé sous forme de deuxième canal de transfert (32.1) s'étend depuis une ouverture au niveau de la deuxième tubulure de raccordement (33.1) jusqu'à une ouverture au niveau de l'aiguille creuse (39), de telle sorte que dans la position de prélèvement de l'élément de perçage (36), l'ouverture du deuxième canal de transfert (32.1) au niveau de la deuxième tubulure de raccordement (33.1) communique avec l'espace interne d'un récipient raccordé, la deuxième tubulure de raccordement (33.1) étant disposée transversalement, notamment essentiellement perpendiculairement, à la direction de pose (H).

10. Dispositif de transfert (20) pour le transfert d'un fluide d'un récipient (17) dans un dispositif de distribution, comprenant un premier adaptateur (1) selon l'une quelconque des revendications 4 à 8, et un deuxième adaptateur (1') selon la revendication 8.

11. Dispositif de transfert (20) selon la revendication 10, **caractérisé en ce que** les accouplements de raccordement des deux adaptateurs (1, 1') sont réalisés de manière complémentaire l'un de l'autre de telle sorte que le premier adaptateur (1) puisse être raccordé de manière étanche aux fluides au deuxième adaptateur (1').

12. Dispositif de transfert (20) selon l'une quelconque des revendications 10 à 11, **caractérisé en ce que** les accouplements de raccordement des deux adaptateurs (1, 1') sont réalisés de telle sorte que les adaptateurs (1, 1') puissent être raccordés l'un à l'autre dans exactement deux orientations, la direction de raccordement (H) du premier adaptateur (1) et la direction d'accouplement (H', M) du deuxième adaptateur (1') étant de préférence opposées dans l'une des deux orientations et étant identiques dans l'autre orientation, les accouplements de raccordement comprenant de préférence des moyens d'encliquetage complémentaires l'un de l'autre (14, 14'), qui prédéfinissent notamment d'une part les orientations des deux adaptateurs (1, 1') et fixent d'autre part de préférence la connexion, établie par les deux accouplements de raccordement, des deux adaptateurs (1, 1').

13. Dispositif de transfert (20) selon l'une quelconque des revendications 10 à 12, **caractérisé en ce que** l'un des accouplements de raccordement des deux adaptateurs (1, 1') comprend un cône externe et l'autre des deux accouplements de raccordement comprend un cône interne, le cône interne étant notamment réalisé au niveau de l'accouplement de raccordement du premier adaptateur (1).

14. Dispositif de transfert selon l'une quelconque des revendications 10 à 13, **caractérisé en ce qu'**au moins un adaptateur supplémentaire (31) est prévu, lequel est réalisé selon la revendication 9, l'accouplement de raccordement de la première tubulure de raccordement (33.2) étant notamment complémentaire de l'accouplement de raccordement du premier adaptateur (1) et de préférence l'accouplement de raccordement de la deuxième tubulure de raccordement (33.1) étant complémentaire de l'accouplement de raccordement du deuxième adaptateur (1'), de telle sorte que l'adaptateur supplémentaire (31) puisse être raccordé simultanément de manière étanche aux fluides directement au premier (1) et au deuxième (1') adaptateur.

15. Dispositif de transfert selon l'une quelconque des revendications 10 à 14, **caractérisé en ce qu'**un boîtier commun est prévu, dans lequel sont disposés les adaptateurs (1, 1') du dispositif de transfert, et éventuellement un dispositif de distribution, dans une configuration prévue pour l'utilisation.
